(19) Europäisches Patentamt
European Patent Office
Office européen des brevets

(11) EP 1 406 858 B1

(12) **EUROPÄISCHE PATENTSCHRIFT**

(45) Veröffentlichungstag und Bekanntmachung des Hinweises auf die Patenterteilung:
**01.07.2009 Patentblatt 2009/27**

(21) Anmeldenummer: **02754882.5**

(22) Anmeldetag: **15.07.2002**

(51) Int Cl.:
*C07C 215/44* (2006.01) *C07D 295/096* (2006.01)
*C07D 209/14* (2006.01) *C07D 333/58* (2006.01)
*C07D 307/81* (2006.01) *C07D 277/66* (2006.01)
*A61K 31/133* (2006.01) *A61P 25/00* (2006.01)

(86) Internationale Anmeldenummer:
**PCT/EP2002/007842**

(87) Internationale Veröffentlichungsnummer:
**WO 2003/008370 (30.01.2003 Gazette 2003/05)**

(54) **SUBSTITUIERTE 4-AMINOCYCLOHEXANOLE**

SUBSTITUTED 4-AMINOCYCLOHEXANOLS

4-AMINOCYCLOHEXANOLS SUBSTITUES

(84) Benannte Vertragsstaaten:
**AT BE BG CH CY CZ DE DK EE ES FI FR GB GR IE IT LI LU MC NL PT SE SK TR**
Benannte Erstreckungsstaaten:
**LT LV RO SI**

(30) Priorität: **17.07.2001 DE 10135636**

(43) Veröffentlichungstag der Anmeldung:
**14.04.2004 Patentblatt 2004/16**

(73) Patentinhaber: **Grünenthal GmbH**
**52078 Aachen (DE)**

(72) Erfinder:
- **SUNDERMANN, Bernd**
**52066 Aachen (DE)**
- **HENNIES, Hagen-Heinrich**
**52152 Simmerath (DE)**
- **ENGLBERGER, Werner**
**52223 Stolberg (DE)**
- **WNENDT, Stephan**
**52066 Aachen (DE)**

(56) Entgegenhaltungen:
**WO-A-01/12195**
**DE-A- 2 839 891**
**DE-A- 19 963 175**

- **F. VONVOIGTLANDER ET AL: "4-(p-Bromophenyl)-4-(dimethylamino)-1-phenethylcyclohexanol, an Extremely Potent Representative of a New Analgesic Series" JOURNAL OF MEDICINAL CHEMISTRY, Bd. 22, Nr. 10, 1979, Seiten 1157-1158, XP002216903 in der Anmeldung erwähnt**
- **KAWAMOTO H ET AL: "Synthesis of J-113397, the first potent and selective ORL1 antagonist" TETRAHEDRON, ELSEVIER SCIENCE PUBLISHERS, AMSTERDAM, NL, Bd. 57, Nr. 6, 4. Februar 2001 (2001-02-04), Seiten 981-986, XP004316527 ISSN: 0040-4020**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 406 858 B1

## Beschreibung

Die vorliegende Erfindung betrifft substituierte 4-Aminocyclohexanole, Verfahren zu deren Herstellung, Arzneimittel enthaltend diese Verbindungen und die Verwendung von substituierten 4-Aminocyclohexanolen zur Herstellung von Arzneimitteln zur Behandlung diverser Indikationen, insbesondere von Schmerz.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist (Mollereau et al., FEBS Letters, 341, 1994, S. 33-38, Darland et al., Trends in Neurosciences, 21, 1998, S. 215-221). Das Peptid ist durch eine hohe Affinität, mit einem $K_d$-Wert von annähernd 56 pM (Ardati et al., Mol. Pharmacol. 51, S. 816-824), und durch eine hohe Selektivität für den ORL1-Rezeptor gekennzeichnet. Der ORL1-Rezeptor ist homolog zu den $\mu$, $\kappa$ und $\delta$ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535). Auch auf der zellulären Ebene sind funktionelle Ähnlichkeiten der $\mu$, $\kappa$ und $\delta$ Opioid-Rezeptoren mit dem ORL1-Rezeptor in Bezug auf die Aktivierung des Kalium-Kanals (Matthes et al., Mol. Pharmacol. 50, 1996, S. 447-450; Vaughan et al., Br. J. Pharmacol. 117, 1996, S. 1609-1611) und der Inhibierung der L-, N- und P/Q-Typ-Kalzium-Kanäle vorhanden (Conner et al., Br. J. Pharmacol. 118, 1996, S. 205-207; Knoflach et al., J. Neuroscience 16, 1996, S. 6657-6664).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794; Hara et al,. Br. J. Pharmacol. 121, 1997, S. 401-408). Diese Befunde können als Hemmung der stressinduzierten Analgesie erklärt werden (Mogil et al., Neurosci. Letters 214, 1996, S131-134; sowie Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin hemmt die Aktivität Kainat- oder Glutamat-stimulierter Hinterwurzelganglienneuronen (Shu et al., Neuropeptides, 32, 1998, 567-571) oder Glutamat-stimulierter Rückenmarksneuronen (Faber et al., Br. J. Pharmacol., 119, 1996, S. 189-190); es wirkt antinociceptiv im Tail Flick-Test in der Maus (King et al., Neurosci. Lett., 223, 1997, 113-116), im Flexor-Reflex-Modell in der Ratte (Xu et al., NeuroReport, 7, 1996, 2092-2094) und im Formalin-Test an der Ratte (Yamamoto et al., Neuroscience, 81, 1997, S. 249-254). In Modellen für neuropathische Schmerzen konnte ebenfalls eine antinociceptive Wirkung von Nociceptin nachgewiesen werden (Yamamoto und Nozaki-Taguchi, Anesthesiology, 87, 1997), die insofern besonders interessant ist, als das die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Sandin et al., Eur. J. Neurosci., 9, 1997, S. 194-197; Manabe et al., Nature, 394, 1997, S. 577-581), Hörvermögen (Nishi et al., EMBO J., 16, 1997, S. 1858-1864), Nahrungsaufnahme (Pomonis et al., NeuroReport, 8, 1996, S. 369-371), Regulation des Blutdruckes (Gumusel et al., Life Sci., 60, 1997, S. 141-145; Campion und Kadowitz, Biochem. Biophys. Res. Comm., 234, 1997, S. 309-312), Epilepsie (Gutiérrez et al, Abstract 536.18, Society for Neuroscience, Vol 24, 28th Ann. Meeting, Los Angeles, November 7.-12, 1998) und Diurese (Kapista et al., Life Sciences, 60, 1997, PL 15-21). In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf $\mu$-Agonisten wie Morphin, Behandlung von Entzugserscheinungen, Reduzierung des Suchtpotentials von Morphinen, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, aterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika aber auch als Antitussiva diskutiert.

Entsprechend vielfältig sind die Anwendungsmöglichkeiten von Verbindungen, die an den ORL1-Rezeptor binden und diesen aktivieren oder inhibieren.

Aufgabe der vorliegenden Erfindung war es, Wirkstoffe zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Ein Gegenstand der Erfindung sind daher nachfolgend als Substanzgruppe A bezeichnete substituierte 4-Aminocyclohexanole gemäß der allgemeinen Formel I,

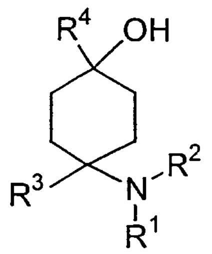

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,

mit $R^5$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus Aryl oder Heteroaryl, jeweils unsubstituiert oder einfach oder mehrfach substituiert;

$R^4$ ausgewählt ist aus $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$

mit Y = O, S oder $H_2$,

mit $R^6$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^7$ ausgewählt aus

H; $C_{3-8}$-Cycloalkyl, Aryl oder Heteroaryl, jeweils unsubstituiert oder einfach oder mehrfach substituiert,

mit $R^8$ ausgewählt aus

Aryl oder Heteroaryl, jeweils unsubstituiert oder einfach oder mehrfach substituiert,

mit L ausgewählt aus

-C(O)-NH-, -NH-C(O)-, -C(O)-O-, -O-C(O)-, -O-, -S- oder $-S(O)_2-$

mit $R^9$ ausgewählt aus

Aryl oder Heteroaryl, jeweils unsubstituiert oder einfach oder mehrfach substituiert,

mit der Maßgabe, daß,

■ (Disclaimer-Gruppe 1) wenn $R^3$ = Phenyl, substituiert oder unsubstituiert, ist und $R^4$ = Phenyl, substituiert oder unsubstituiert, oder $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$

mit Y = $H_2$,

$R^6$ = H, $C_{1-5}$-Alkyl, gesättigt oder ungesättigt, verzweigt oder unverzweigt, einfach oder mehrfach substituiert oder unsubstituiert

und/oder

$R^7$ = H, $C_{3-8}$-Cycloalkyl oder Phenyl, jeweils substituiert oder unsubstituiert, bedeuten,

$R^1$ und $R^2$ nicht beide unabhängig voneinander $C_{1-5}$-Alkyl sind,

(Disclaimer-Gruppe 2) wenn $R^3$ = Thiophenyl, substituiert oder unsubstituiert, und $R^4$ = $-CH_2-CH_2$-Phenyl, die Reste $R^1$ und $R^2$ nicht zusammen einen Ring bilden und $(CH_2)_5$ bedeuten,

wobei "Aryl oder Heteroaryl einfach oder mehrfach substituiert" für "Aryl oder Heteroaryl einfach oder mehrfach substituiert mit $R^{82}$, $OR^{82}$, Halogen, $CF_3$, CN, $NO_2$, $NR^{83}R^{84}$, $C_{1-6}$-Alkyl, $C_{1-6}$-Alkoxy, $C_{3-8}$-Cycloalkoxy, $C_{3-8}$-Cycloalkyl oder $C_{2-6}$-Alkylen" steht
wobei
$R^{62}$ für H, $C_{1-10}$-Alkyl-, Aryl, Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest,
wobei diese Aryl und Heteroarylreste nicht selbst mit Aryl- oder Heteroaryl-Resten substituiert sein dürfen, steht;
$R^{83}$ und $R^{84}$ gleich oder verschieden, H, $C_{1-10}$-Alkyl, Aryl, Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste $R^{83}$ und $R^{84}$ zusammen $CH_2CH_2OCH_2CH_2$,
$CH_2CH_2NR^{85}CH_2CH_2$ oder $(CH_2)_{3-6}$ bedeuten, und
der Rest $R^{85}$ für H, $C_{1-10}$-Alkyl, Aryl, Heteroaryl oder für einen über $C_{1-3}$-Alkyl, gesättigt oder ungesättigt, oder eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen;
"Cycloalkyl, einfach oder mehrfach substituiert" für "Cycloalkyl, einfach oder mehrfach substituiert mit F, Cl, Br, I, $NH_2$, SH, OH, $OC_{1-3}$-Alkyl oder $C_{1-3}$-Alkyl" steht
und
"Alkyl, einfach oder mehrfach substituiert" für "Alkyl, einfach oder mehrfach substituiert mit F, Cl, Br, I, $NH_2$, SH oder OH" steht,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Alle diese erfindungsgemäßen Verbindungen bzw. Verbindungsgruppen zeigen hervorragende Bindung an den ORL1-Rezeptor.

Verbindungen, die eine gewisse entfernte strukturelle Verwandtschaft mit den hier vorgeschlagenen Verbindungen zeigen, sind aus folgenden Schriften bekannt:

Der DE-OS-28 39 891 bzw. dem parallelen US-Patent US 4,366,172 (Lednicer et al.). Darin werden die genannten Verbindungen, als analgetisch wirksam beschrieben, ohne daß Bezug auf den ORL1-Rezeptor genommen wird. Den parallelen Artikeln:

D. Lednicer und P.F. von Voightlander, J. Med. Chem. 1979, 22, 1157,

- D. Lednicer, P.F. von Voightlander und D.E. Emmert, J. Med. Chem. 1980,23,424,und
- D. Lednicer, P.F. von Voightlander und D.E. Emmert, J. Med. Chem. 1981,24,404,
- D. Lednicer, P.F. von Voightlander und D.E. Emmert, J. Med. Chem. 1981,24,340,
- P.F. VonVoightlander, D. Lednicer, R.A. Lewis und D.D. Gay, "Endogenous and Exogenous Opiate Agonists and Antagonists", Proc. Int. Narc. Res. Club Conf. (1980), Meeting Date 1979, Way E.Long (Ed), Publisher: Pergamon, Elmsford, N.Y.International, Pergamon, 1980, 17-21,

■ Kamenka et al., EurJ.Med.Chem.Chim.Ther.; FR; 19;3;1984;255-260 und
■ Rao M.N.A. und Rao S.C. Indian Drugs, 1985, 22 (5), 252-257.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1 - oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-6}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyl, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, - $CH_2-CH_2-$, $-CH_2-CH_2-CH_2-$ und $-CH_2-CH_2-CH_2-CH_2-$ zu verstehen, unter $(CH_2)_{4-5}$ ist - $CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{82}$, $OR^{82}$ einem Halogen, vorzugsweise F und/oder Cl, einem $CF_3$, einem CN, einem $NO_2$, einem $NR^{83}R^{84}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{82}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, die Reste $R^{83}$ und $R^{84}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{83}$ und $R^{84}$ bedeuten zusammen $CH_2CH_2OCH_2CH_2$,

$CH_2CH_2NR^{85}CH_2CH_2$ oder $(CH_2)_{3-6}$, und der Rest $R^{85}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter physiologisch verträglich ist zu verstehen, daß die Substanz, insbesondere das Salz als solches, bei Anwendung im Menschen oder Säugetier verträglich ist, also beispielsweise nicht unphysiologisch (z.B. giftig) wirkt.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/ oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele

für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetierveträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

Ein weiterer Gegenstand der Erfindung sind substituierte 4-Aminocyclohexanole der allgemeinen Formel I, , worin die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten (Substanzgruppe B).

Ein weiterer Gegenstand der Erfindung sind substituierte 4-Aminocyclohexanole der allgemeinen Formel I, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen (Substanzgruppe C).

Ein weiterer Gegenstand der Erfindung sind substituierte 4-Aminocyclohexanole der allgemeinen Formel I, , worin

$R^3$ ausgewählt ist aus Heteroaryl, jeweils unsubstituiert oder einfach oder mehrfach substituiert (Substanzgruppe D).

Ein weiterer Gegenstand der Erfindung sind substituierte 4-Aminocyclohexanole der allgemeinen Formel I, , worin

$R^3$ ausgewählt ist aus Aryl, unsubstituiert oder einfach oder mehrfach substituiert (Substanzgruppe E).

Ein weiterer Gegenstand der Erfindung sind substituierte 4-Aminocyclohexanole der allgemeinen Formel I, worin

die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,

$R^3$ ausgewählt ist aus Aryl, jeweils unsubstituiert oder einfach oder mehrfach substituiert (Substanzgruppe F).

Ein weiterer Gegenstand der Erfindung sind substituierte 4-Aminocyclohexanole der allgemeinen Formel I, , worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,

$R^3$ ausgewählt ist aus Heteroaryl, unsubstituiert oder einfach oder mehrfach substituiert (Substanzgruppe G).

Ein weiterer Gegenstand der Erfindung sind substituierte 4-Aminocyclohexanole der allgemeinen Formel I, worin

$R^7$ ausgewählt ist aus

Heteroaryl, jeweils unsubstituiert oder einfach oder mehrfach substituiert (Substanzgruppe H).

Ein weiterer Gegenstand der Erfindung sind substituierte 4-Aminocyclohexanole der allgemeinen Formel I,

, worin
$R^4$ ausgewählt ist aus $-CHR^6R^7$, $-CHR^6- CH_2R^7$, $-CHR^6-CH_2-CH_2R^7$ oder $-CHR^6-CH_2-CH_2-CH_2R^7$
mit $R^6$ ausgewählt aus
$C(O)O-C_{1-6}$-Alkyl, gesättigt oder ungesättigt, verzweigt oder unverzweigt, einfach oder mehrfach substituiert oder unsubstituiert (Substanzgruppe J).

Ein weiterer Gegenstand der Erfindung sind substituierte 4-Aminocyclohexanole der allgemeinen Formel I,
, worin
$R^4$ ausgewählt ist aus $-C(Y)R^7$, $-C(Y)-CH_2R^7$, $-C(Y)-CH_2-CH_2R^7$ oder $- C(Y)-CH_2-CH_2-CH_2R^7$
mit Y = O oder S (Substanzgruppe K).

Bezüglich der Substanzgruppen A, D, E, H, J oder K ist es dabei bevorzugt, wenn
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,
mit $R^5$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

Bezüglich der Substanzgruppen C oder G ist es dabei bevorzugt, wenn
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
insbesondere
$R^1$ und $R^2$ unabhängig voneinander ausgewählt sind aus Methyl oder Ethyl.

Bezüglich der Substanzgruppen B oder F ist es dabei bevorzugt, wenn
$R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,
mit $R^5$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

Bezüglich der Substanzgruppen A, B, C, H, J oder K ist es dabei bevorzugt, wenn
$R^3$ ausgewählt ist aus Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
vorzugsweise
$R^3$ ausgewählt ist aus Phenyl, Thiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Pyridyl, Furyl oder Thiophenyl, jeweils unsubstituiert oder einfach oder mehrfach substituiert.

Bezüglich der Substanzgruppen D oder G ist es dabei bevorzugt, wenn
$R^3$ ausgewählt ist aus Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
vorzugsweise ,
$R^3$ ausgewählt ist aus Thiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Pyridyl, Furyl oder Thiophenyl, jeweils unsubstituiert oder einfach oder mehrfach substituiert.

Bezüglich der Substanzgruppen E oder F ist es dabei bevorzugt, wenn

$R^3$ ausgewählt ist aus Phenyl, Naphthyl, Anthracenyl;
vorzugsweise
$R^3$ ausgewählt ist aus Phenyl oder Naphthyl, jeweils unsubstituiert oder einfach oder mehrfach substituiert;
insbesondere
$R^3$ ausgewählt ist aus Phenyl, unsubstituiert oder einfach oder mehrfach substituiert.

Bezüglich der Substanzgruppen A bis G ist es dabei bevorzugt, wenn
$R^4$ ausgewählt ist aus $C_{3-8}$-Cycloalkyl, Aryl oder Heteroaryl, jeweils unsubstituiert oder einfach oder mehrfach substituiert; oder $-R^8-L-R^9$
vorzugsweise
$R^4$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert; ; oder $-R^8-L-R^9$,
insbesondere
$R^4$ ausgewählt ist aus Cyclopentyl, Cyclohexyl, Cycloheptyl, Cyclooctyl, Anthracenyl, Indolyl, Naphthyl, Benzofuranyl, Benzothiazolyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert; oder $-R^8-L-R^9$.

Bezüglich der vorstehenden Ausführungsform ist es dann auch bevorzugt, wenn
$R^8$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
L ausgewählt aus
-C(O)-NH-, -NH-C(O)-, -C(O)-O-, -O-C(O)-, -O-, -S- oder-$S(O)_2$-,
und/oder $R^9$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
vorzugsweise
$R^8$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert,
L ausgewählt aus
-C(O)-NH-, -NH-C(O)-, -C(O)-O-, -O-C(O)- oder -$S(O)_2$-,
und/oder $R^9$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert
insbesondere
$R^8$ ausgewählt ist aus
Indolyl, unubstituiert,
L ausgewählt aus

$$-S(O)_2-$$

und $R^9$ ausgewählt ist aus
Phenyl unsubstituiert.

Bezüglich der Substanzgruppen A bis G ist es ebenfalls eine bevorzugte Ausführungsform, wenn
$R^4$ ausgewählt ist aus $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder-$C(Y)-CH_2-CH_2-CH_2R^7$
mit Y = O, S oder $H_2$,
vorzugsweise

$R^4$ ausgewählt ist aus $-CHR^6R^7$, $-CHR^6- CH_2R^7$, $-CHR^6-CH_2-CH_2R^7$, $- C(Y)R^7$, $-C(Y)-CH_2R^7$ oder $-C(Y)-CH_2-CH_2R^7$ mit Y = O oder S,
insbesondere
$R^4$ ausgewählt ist aus $-CHR^6R^7$, $-CHR^6- CH_2R^7$, $-C(Y)R^7$ oder $-C(Y)-CH_2R^7$ mit Y = O.

Bezüglich der vorstehenden Ausführungsform ist es dann auch bevorzugt, wenn
$R^6$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert;
insbesondere
H, $CH_3$ und $C_2H_5$.

Und es ist bezüglich der vorstehenden Ausführungsform dann auch bevorzugt, wenn
$R^7$ ausgewählt ist aus $C_{3-8}$-Cycloalkyl, Aryl oder Heteroaryl, jeweils unsubstituiert oder einfach oder mehrfach substituiert;
vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Bezüglich der Substanzgruppe H ist es eine bevorzugte Ausführungsform, wenn
$R^4$ ausgewählt ist aus Heteroaryl, jeweils unsubstituiert oder einfach oder mehrfach substituiert; oder $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder-$C(Y)-CH_2-CH_2-CH_2R^7$; oder $-R^8-L-R^9$
mit Y = $H_2$,
vorzugsweise
$R^4$ ausgewählt ist aus Indolyl, Benzofuranyl, Benzothiophenyl, Indanyl, Benzodioxanyl, Benzodioxolanyl, Acenaphthyl, Carbazolyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert; oder $-CHR^6R^7$, $-CHR^6-CH_2R^7$, $-CHR^6-CH_2-CH_2R^7$, $-C(Y)R^7$, $- C(Y)-CH_2R^7$ oder $-C(Y)-CH_2-CH_2R^7$; oder $-R^8-L-R^9$
mit Y = $H_2$,
insbesondere
$R^4$ ausgewählt ist aus Indolyl, Benzofuranyl, Benzothiophenyl, Indanyl, Benzodioxanyl, Benzodioxolanyl, Acenaphthyl, Carbazolyl, Thiophenyl, Benzothiazolyl, Furyl, Pyridyl, Pyrrolyl, Pyrazinyl oder Pyrimidyl, jeweils unsubstituiert oder einfach oder mehrfach substituiert; oder $-CHR^6R^7$, $- CHR^6-CH_2R^7$, $-C(Y)R^7$ oder $-C(Y)-CH_2R^7$, ; oder $-R^8-L-R^9$
mit Y = $H_2$.

Bezüglich der vorstehenden Ausführungsform ist es dann auch bevorzugt, wenn
$R^6$ ausgewählt ist aus
H, $C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert;
insbesondere
H, $CH_3$ und $C_2H_5$
und/oder
$R^7$ ausgewählt ist aus Indolyl, Benzofuranyl, Benzothiophenyl, Indanyl, Benzodioxanyl, Benzodioxolanyl, Acenaphthyl, Carbazolyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert;
vorzugsweise
$R^7$ ausgewählt ist aus Indolyl, Benzofuranyl, Benzothiophenyl, Indanyl, Benzodioxanyl, Benzodioxolanyl, Acenaphthyl, Carbazolyl, Thiophenyl, Furyl, Pyridyl, Pyrrolyl, Pyrazinyl oder Pyrimidyl, jeweils unsubstituiert oder einfach oder mehrfach substituiert;
insbesondere
$R^7$ ausgewählt ist aus Thiophenyl, Furyl, Pyridyl, Pyrrolyl, Pyrazinyl oder Pyrimidyl, jeweils unsubstituiert oder einfach oder mehrfach substituiert.

Und es ist weiter bezüglich der vorstehenden Ausführungsform dann auch bevorzugt, wenn
$R^8$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
L ausgewählt aus
-C(O)-NH-, -NH-C(O)-, -C(O)-O-, -O-C(O)-, -O-, -S- oder $-S(O)_2-$,
und/oder $R^9$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
vorzugsweise
$R^8$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert,
L ausgewählt aus
-C(O)-NH-, -NH-C(O)-, -C(O)-O-, -O-C(O)- oder $-S(O)_2-$,
und/oder $R^9$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert
insbesondere
$R^8$ ausgewählt ist aus
Indolyl, unubstituiert,
L ausgewählt aus

$-S(O)_2-$

und $R^9$ ausgewählt ist aus
Phenyl unsubstituiert.

Bezüglich der Substanzgruppe J ist es eine bevorzugte Ausführungsform, wenn
$R^4$ ausgewählt ist aus $-CHR^6R^7$, $-CHR^6-CH_2R^7$ oder $-CHR-CH_2-CH_2R^7$
vorzugsweise
$R^4$ ausgewählt ist aus $-CHR^6R^7$ oder $-CHR^6-CH_2R^7$,
insbesondere
$R^4$ ausgewählt ist aus $-CHR^6R^7$.

Bezüglich der vorstehenden Ausführungsform ist es dann auch bevorzugt, wenn
$R^6$ ausgewählt ist aus
$C(O)O-C_{1-4}$-Alkyl, gesättigt oder ungesättigt, verzweigt oder unverzweigt, einfach oder mehrfach substituiert oder unsubstituiert;
vorzugsweise
$C(O)O-C_{1-3}$-Alkyl, gesättigt oder ungesättigt, verzweigt oder unverzweigt, einfach oder mehrfach substituiert oder unsubstituiert;
insbesondere
$C(O)O-CH_3$ und $C(O)O-C_2H_5$

und/oder
$R^7$ ausgewählt ist aus $C_{3-8}$-Cycloalkyl, Aryl oder Heteroaryl, jeweils unsubstituiert oder einfach oder mehrfach substituiert;
vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Bezüglich der Substanzgruppe K ist es eine bevorzugte Ausführungsform, wenn
$R^4$ ausgewählt ist aus $-C(Y)R^7$, $-C(Y)-CH_2R^7$, $-C(Y)-CH_2-CH_2R^7$ oder-$C(Y)-CH_2-CH_2-CH_2R^7$
mit Y = O
vorzugsweise
$R^4$ ausgewählt ist aus $-C(Y)R^7$, $-C(Y)-CH_2R^7$ oder $-C(Y)-CH_2-CH_2R^7$,
mit Y = O
insbesondere
$R^4$ ausgewählt ist aus $-C(Y)R^7$ oder $-C(Y)-CH_2R^7$,
mit Y = O.

Bezüglich der vorstehenden Ausführungsform ist es dann auch bevorzugt, wenn
$R^7$ ausgewählt ist aus $C_{3-8}$-Cycloalkyl, Aryl oder Heteroaryl, jeweils unsubstituiert oder einfach oder mehrfach substituiert;
vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzatriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

In einer bevorzugten Ausführungsform aller vorstehend aufgeführten Substanzgruppen sind die erfindungsgemäßen substituierten 4-Aminocyclohexanole ausgewählt aus der folgenden Gruppe:
4-Dimethylamino-1-(1-methyl-1H-indol-2-yl)-4-phenylcyclohexanol 1-Benzo[b]thiophen-2-yl-4-dimethylamino-4-phenylcyclohexanol 1-Benzo[b]thiophen-3-yl-4-dimethylamino-4-phenylcyclohexanol 1-(1-Benzolsulfonyl-1H-indol-2-yl)-4-dimethylamino-4-phenylcyclohexanol 1-Benzofuran-2-yl-4-dimethylamino-4-phenylcyclohexanol

- 1-Benzothiazol-2-yl-4-dimethylamino-4-phenylcyclohexanol
  gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Die erfindungsgemäßen Substanzen sind toxikologisch unbedenklich, so daß sie sich als pharmazeutischer Wirkstoff in Arzneimittel eignen.

Ein weiterer Gegenstand der Erfindung sind daher Arzneimittel enthaltend wenigstens ein erfindungsgemäßes substituiertes 4-Aminocyclohexanol gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten

4-Aminocyclohexanol gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte 4-Amino-Cyclohexanolderivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten 4-Aminocyclohexanole verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen substituierten 4-Aminocyclohexanols appliziert.

Für alle vorstehenden Formen der erfindungsgemäßen Arzneimittel ist es besonders bevorzugt, wenn das Arzneimittel neben wenigstens einem substituierten 4-Aminocyclohexanol noch ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes 4-Aminocyclohexanol als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße substituierte 4-Amino-Cyclohexanolderivate zur Herstellung eines Arzneimittels zur Behandlung von Schmerz, insbesondere von akutem, viszeralem, neuropathischem oder chronischem Schmerz, verwendet werden.

Ein weiterer Gegenstand der Erfindung ist daher die Verwendung eines erfindungsgemäßen substituierten 4-Aminocyclohexanols gemäß der allgemeinen Formel I gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; zur Herstellung eines Arzneimittels zur Behandlung von Schmerz, insbesondere von akutem, viszeralem, neuropathischem oder chronischem Schmerz.

Wie bereits in der Einleitung ausgeführt, spielt der ORL1-Rezeptor neben der Funktion im Schmerzgeschehen noch in einer Vielzahl anderer physiologischer Prozeße insbesondere von medizinisch relevanter Bedeutung eine Rolle.

Daher ist ein weiterer Gegenstand der Erfindung die Verwendung eines substituierten 4-Aminocyclohexanols der allgemeinen Formel I,

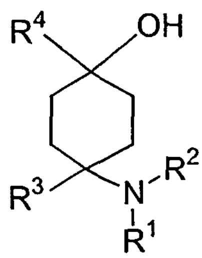

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,

mit $R^5$ ausgewählt aus H; $C_{1-8}$-Alkyl oder $C_{3-8}$-Cycloalkyl, jeweils gesättigt oder ungesättigt, verzweigt oder unverzweigt,

einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus Aryl oder Heteroaryl, jeweils unsubstituiert oder einfach oder mehrfach substituiert;

$R^4$ ausgewählt ist aus $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$

mit Y = O, S oder $H_2$,

mit $R^6$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^7$ ausgewählt aus

H; $C_{3-8}$-Cycloalkyl, Aryl oder Heteroaryl, jeweils unsubstituiert oder einfach oder mehrfach substituiert,

mit $R^8$ ausgewählt aus

Aryl oder Heteroaryl, jeweils unsubstituiert oder einfach oder mehrfach substituiert,

mit L ausgewählt aus

-C(O)-NH-, -NH-C(O)-, -C(O)-O-, -O-C(O)-, -O-, -S- oder $-S(O)_2-$

mit $R^9$ ausgewählt aus

Aryl oder Heteroaryl, jeweils unsubstituiert oder einfach oder mehrfach substituiert,

wobei "Aryl oder Heteroaryl einfach oder mehrfach substituiert" für "Aryl oder Heteroaryl, einfach oder mehrfach substituiert mit $R^{82}$, $OR^{82}$, Halogen, $CF_3$, CN, $NO_2$, $NR^{83}R^{84}$, $C_{1-6}$-Alkyl, $C_{1-6}$-Alkoxy, $C_{3-8}$-Cycloalkoxy, $C_{3-8}$-Cycloalkyl oder $C_{2-6}$-Alkylen" steht

wobei

$R^{82}$ für H, $C_{1-10}$-Alkyl, Aryl Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest,

wobei diese Aryl und Heteroarylreste nicht selbst mit Aryl- oder Heteroaryl-Resten substituiert sein dürfen, steht;

$R^{83}$ und $R^{84}$ gleich oder verschieden, H, $C_{1-10}$-Alkyl, Aryl, Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen, oder die Reste $R^{83}$ und $R^{84}$ zusammen $CH_2CH_2OCH_2CH_2$, $CH_2CH_2NR^{85}CH_2CH_2$ oder $(CH_2)_{3-6}$ bedeuten, und

der Rest $R^{85}$ für H, $C_{1-10}$-Alkyl, Aryl, oder Heteroaryl oder für einen über $C_{1-3}$-Alkyl, gesättigt oder ungesättigt, oder eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen;

"Cycloalkyl, einfach oder mehrfach substituiert" für "Cycloalkyl, einfach oder mehrfach substituiert mit F, Cl, Br, I, $NH_2$, SH, OH, $OC_{1-3}$-Alkyl oder $C_{1-3}$-Alkyl" steht

und

"Alkyl, einfach oder mehrfach substituiert" für "Alkyl, einfach oder mehrfach substituiert mit F, Cl, Br, I, $NH_2$, SH oder OH" steht,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum, Antitussivum oder Anesthetikum bzw. zur Coadministration bei Behandlung mit einem opioiden Analgetikum oder mit einem Anesthetikum, zur Diurese oder Antinatriurese und/oder Anxiolyse.

Dabei kann es in einer der vorstehenden Verwendungen bevorzugt sein, wenn ein verwendetes substituiertes 4-Aminocyclohexanol als reines Diastereomer und/oder Enantiomer, als Razemat oder als nicht-äquimolare oder äquimolare Mischung der Diastereomere und/oder Enantiomere vorliegt und/oder neben dem substituierten 4-Aminocyclohexanol noch ein Opioid, vorzugsweise ein starkes Opioid, insbesondere Morphin, oder ein Anesthetikum, vorzugsweise Hexobarbital oder Halothan, verwendet wird.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen,

insbesondere chronischer Schmerzen, benötigt, durch Verabreichung einer therapeutisch wiksamen Dosis eines erfindungsgemäßen substituierten 4-Aminocyclohexanols oder eines erfindungsgemäßen Arzneimittels.

Ein weiterer Gegenstand der Erfindung ist ein Verfahren zur Herstellung der erfindungsgemäßen substituierten 4-Aminocyclohexanole wie in der folgenden Beschreibung und Beispielen ausgeführt.

Insbesondere geeignet ist dabei ein Verfahren mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

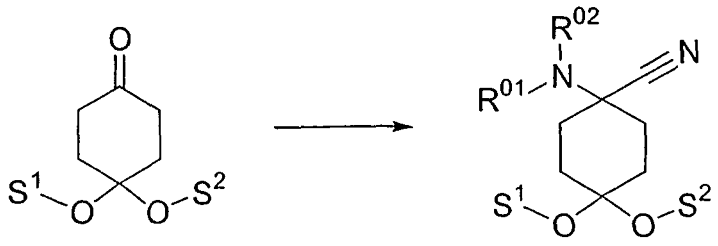

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit metallorganischen Reagenzien, bevorzugt Grignard- oder Organolithiumreagenzien, der Formel Metall-$R^3$ umgesetzt, so daß eine Verbindung gemäß Formel IVa entsteht;

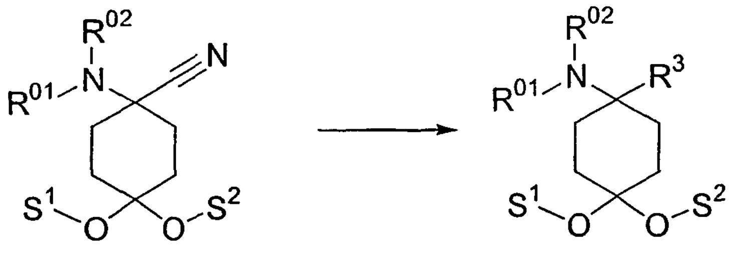

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa gemäß Formel III werden die Schutzgruppen $S^1$ und $S^2$ abgespalten, so daß ein 4-substituiertes 4-Aminocyclohexanonderivat gemäß Formel IV entsteht;

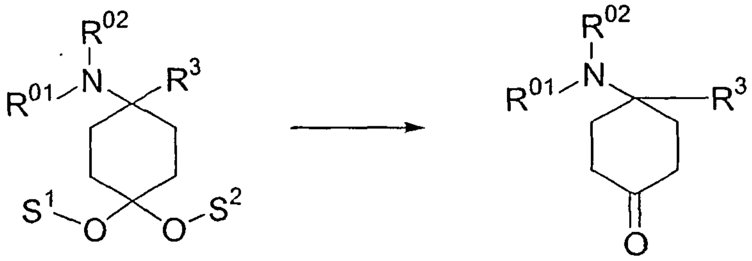

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IV mit metallorganischen Reagenzien, bevorzugt Grignard- oder Organolithiumreagenzien, der Formel Metall-$R^{04}$ umgesetzt, so daß eine Verbindung gemäß Formel V entsteht;

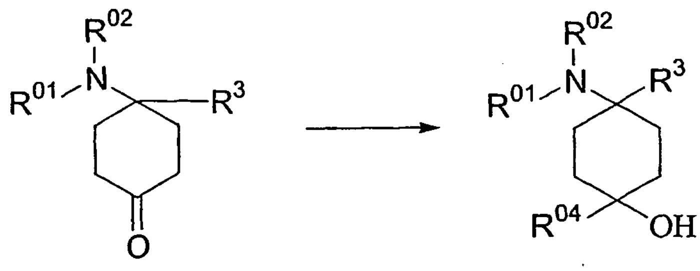

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$ und $R^4$ die in Anspruch 1 angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; oder die Reste $R^{01}$ und $R^{02}$ zusammen einen Ring bilden und $CH_2CH_2OCH_2CH_2$, $CH_2CH_2NR^{05}CH_2CH_2$ oder $(CH_2)_{3-6}$ bedeuten,
mit $R^{05}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, -

$CHR^6$-$CH_2$-$CH_2$-$CH_2R^7$, -C(Y)$R^7$, -C(Y)-$CH_2R^7$, -C(Y)-$CH_2$-$CH_2R^7$ oder - C(Y)-$CH_2$-$CH_2$-$CH_2R^7$; oder -$R^8$-L-$R^9$
mit Y = O, S oder $H_2$,
mit $R^6$ ausgewählt aus
H, $C_{1-7}$-Alkyl, gesättigt oder ungesättigt, verzweigt oder unverzweigt, einfach oder mehrfach substituiert oder unsubstituiert;
und mit $R^7$ ausgewählt aus
H; $C_{3-8}$-Cycloalkyl, Aryl oder Heteroaryl, jeweils unsubstituiert oder einfach oder mehrfach substituiert,
mit $R^8$ ausgewählt aus
Aryl oder Heteroaryl, jeweils unsubstituiert oder einfach oder mehrfach substituiert,
mit L ausgewählt aus
-C(O)-NH-, -NH-C(O)-, -C(O)-O-, -O-C(O)-, -O-, -S- oder -$S(O)_2$-
mit $R^9$ ausgewählt aus
Aryl oder Heteroaryl, jeweils unsubstituiert oder einfach oder mehrfach substituiert,
und $S^1$ und $S^2$ unabhängig voneinander ausgewählt sind aus Schutzgruppen oder zusammen eine Schutzgruppe bedeuten, vorzugsweise Monoacetal.

Besonders bevorzugt ist es bei vorstehenden Verfahren, wenn die Schutzgruppen am H bei $R^{01}$, $R^{02}$, $R^{04}$ und/oder $R^{05}$ ausgewählt sind aus Alkyl, Benzyl oder Carbamaten, beispielsweise FMOC, Z oder Boc.

**Beispiele**

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder synthetisiert.

Die Analytik erfolgte über NMR-Spektroskopie, gegebenenfalls in Kombination mit anderen analytischen Verfahren wie Dünnschichtchromatographie, Massenspektrometrie oder HPLC.

**Beispiel 1**

Allgemeine Möglichkeit der Herstellung der Verbindungen

Die Herstellung dieser Verbindungen erfolgt ausgehend von einem geeignet als beispielsweise Monoacetal geschützten Cyclohexan-1,4-dion II. Durch Umsetzung mit Kaliumcyanid in Gegenwart eines sekundären Amins wird ein geschütztes N-substituiertes 1-Amino-4-oxo-cyclohexancarbonitrilderivat III erhalten.

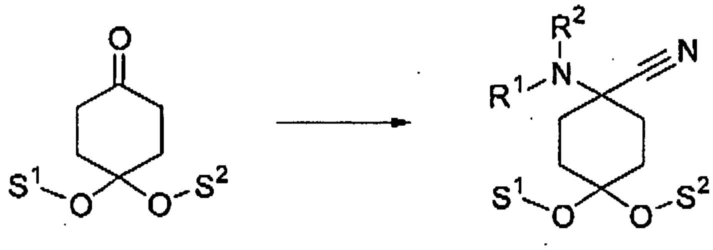

II III

Die Umsetzung des Aminonitrils III mit metallorganischen Reagenzien, bevorzugt Grignard- oder Organolithiumreagenzien, bewirkt eine Substitution der Nitrilfunktion, so daß nach anschließender Abspaltung der Carbonylschutzgruppe ein 4- substituiertes 4-Aminocyclohexanonderivat IV erhalten wird.

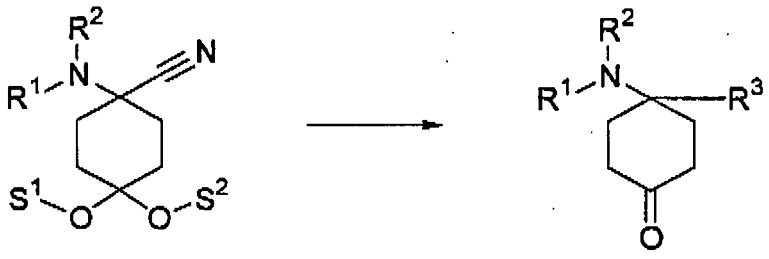

III IV

Intermediate des Typs IV können schließlich durch Addition metallorganischer Reagenzien, bevorzugt Grignard- oder Organolithiumreagenzien, in 4-Aminocyclohexanole I überführt werden.

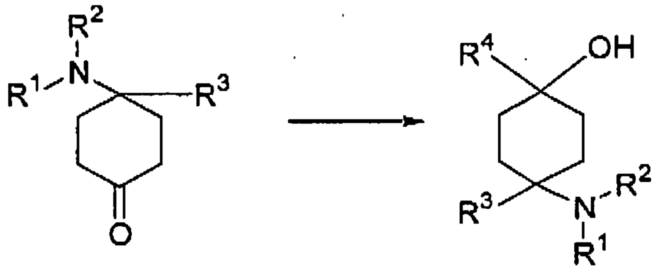

IV I

**Beispiel 2**

Messung der ORL1-Bindung

Die 4-Aminocyclohexanole der allgemeinen Formel I wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM.

Die Bindungsassays wurden mit je 20 $\mu$g Membranprotein je 200 $\mu$l Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird als $K_i$-Wert angegeben.

Von jedem der nachfolgenden Beispiele 4 bis 12 wurde gemäß den angegebenen molekularpharmakologischen Untersuchungen die Affinität zum ORL1-Rezeptor bestimmt. Die entsprechenden Ki-Werte sind in der nachfolgenden Tabelle 1 angegeben.

Tabelle 1: Daten des ORL1-Bindungsassays

| Beispiel | | Ki (nM) |
|---|---|---|
| 4 | | 4,4 |
| 5 | | 10 |
| 6 | | 9,0 |
| 7 | | 24 |
| 8 | | 7,2 |

(fortgesetzt)

| Beispiel | | Ki (nM) |
|---|---|---|
| 9 | | 3,4 |
| 10 | | 110 |
| 11 | | 66 |
| 12 | | 430 |

**Beispiel 3**

Analgesieprüfung im Tail-Flick Test an der Maus

Die analgetische Wirksamkeit der Verbindungen wurde im Brennstrahl (Tail-flick) Test an der Maus nach der Methode von D'Amour and Smith (J. Pharm. Exp. Ther. 72, 74 79 (1941) untersucht. Dazu wurden NMRI-Mäuse mit einem Gewicht zwischen 20 - 24 g verwendet. Die Tiere wurden einzeln in spezielle Testkäfige gesetzt und die Schwanzbasis einem focussierten Wärmestrahl einer elektrischen Lampe (Tail-flick Typ 55/12/10.fl, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 3 - 5 Sekunden betrug. Vor Gabe einer Verbindung wurden die Tiere innerhalb von fünf Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/(T_2 - T_0)] \times 100$$

Dabei ist die $T_0$ die Latenzzeit vor und $T_1$ die Latenzzeit nach Substanzapplikation, $T_2$ ist die maximale Expositionszeit (12 sec).
Zur Bestimmung der Dosisabhängigkeit wurde die jeweilige Verbindung in 3 - 5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum 20 Minuten nach intravenöser Substanzgabe.
Die untersuchten Verbindungen zeigten eine ausgeprägte analgetische Wirkung. Die Ergebnisse sind in der nachfolgenden Tabelle zusammengefaßt.

| **Beispiel** | | **% MPE** | **$ED_{50}$** |
|---|---|---|---|
| Nr. | | (Dosierung in mg/kg intravenös) | mg/kg intravenös |
| 4 | | 100 (0,1) | 0,009 |
| 5 | | 35 (0,001)<br>79 (0,01) | 0,01 - 0,001 |
| 6 | | 43 (0,001)<br>96 (0,01) | 0,001 |
| 7 | | 100 (1) | 0,19 |
| 9 | | 100 (1) | |

**Beispiel 4**

4-Dimethylamino-1-phenethyl-4-phenylcyclohexanol

200 g 1,4-Dioxaspiro[4.5]decan-8-on wurden vorgelegt, nacheinander 1,68 l wässrige Dimethylaminlösung (40 Volumenprozent), 200 ml Methanol, 200 g Kaliumcyanid und 303 g Dimethylamin Hydrochlorid zugegeben und die Reaktionsmischung für 65 Stunden bei Raumtemperatur gerührt. Die erhaltene weiße Suspension wurde viermal mit je

800 ml Diethylether extrahiert, die vereinigten Extrakte zunächst eingeengt und mit 500 ml Dichlormethan aufgenommen, die organische Phase abgetrennt, über Natriumsulfat getrocknet, filtriert, eingeengt und im Vakuum weitgehend von Lösungsmittelresten befreit. Es wurden 265 g 8-Dimethylamino-1,4-dioxaspiro[4.5]decan-8-carbonitril als weißer Feststoff erhalten.
30 g 8-Dimethylamino-1,4-dioxaspiro[4.5]decan-8-carbonitril wurden in 300 ml Tetrahydrofuran p.a. gelöst, unter Stickstoffatmosphäre 143 ml 2,0 molare Phenylmagnesiumchloridlösung in THF zugegeben und über Nacht bei Raumtemperatur gerührt. Zur Aufarbeitung wurden unter Eiskühlung 100 ml Ammoniumchloridlösung (20 Massenprozent) zugegeben, die Phasen getrennt, die wässrige Phase zweimal mit je 250 ml Diethylether extrahiert, die vereinigten organischen Phasen über Natriumsulfat getrocknet, filtriert, eingeengt und im Vakuum weitgehend von Lösungsmittelresten befreit.
Das erhaltene rohe Dimethyl-(8-phenyl-1,4-dioxaspiro[4.5]dec-8-yl)amin (34,5 g) wurde ohne weitere Aufreinigung für 48 Stunden mit einem Gemisch aus 83 ml konz. Salzsäure (32 Massenprozent) und 48 ml Wasser bei Raumtemperatur gerührt. Anschließend wurde die Reaktionsmischung zunächst dreimal mit je 50 ml Diethylether gewaschen, dann unter Eiskühlung durch Zugabe von 100 ml Natronlauge (32 Massenprozent) alkalisiert, dreimal mit je 100 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 18,8 g 4-Dimethylamino-4-phenylcyclohexanon erhalten. 21 ml 1,0 molare Phenethylmagnesiumchloridlösung in Tetrahydrofuran wurden vorgelegt und 3,83 g 4-Dimethylamino-4-phenylcyclohexanon, gelöst in 10 ml Tetrahydrofuran, zugetropft und über Nacht bei Raumtemperatur gerührt. Zur Aufarbeitung wurden unter Eiskühlung 15 ml Ammoniumchloridlösung (20 Massenprozent) zugegeben, dreimal mit je 15 ml Ethylacetat extrahiert, die Extrakte vereinigt, über Natriumsulfat getrocknet, filtriert, eingeengt und im Vakuum weitgehend von Lösungsmittelresten befreit. Das erhaltene rohe 4-Dimethylamino-1-phenethyl-4-phenylcyclohexanol (5,75 g) als braunes Öl erhalten, das mit Diethylether/Hexan (V:V = 1:1) an Kieselgel chromatographiert wurde. Es wurden 1,71 g 4-Dimethylamino-1-phenethyl-4-phenylcyclohexanol als gelbe Kristalle erhalten, die, in 6,8 ml 2-Butanon gelöst, durch Umsetzung mit 26μl Wasser und 366 μl Chlortrimethylsilan, Rühren über Nacht mit anschließender Filtration in 838 mg des korrespondierenden Hydrochlorids überführt wurden.

**Beispiel 5**

4-(4-Chlorphenyl)-4-dimethylamino-1-phenethylcyclohexanol

38 ml 1,0 molare 4-Chlorphenylmagensiumchloridlösung in Diethylether wurden vorgelegt und 4,00 g 8-Dimethylamino-1,4-dioxaspiro[4.5]decan-8-carbonitril, gelöst in 60 ml Diethylether, zugetropft und über Nacht bei Raumtemperatur gerührt. Zur Aufarbeitung wurden unter Eiskühlung 30 ml Ammoniumchloridlösung (20 Massenprozent) zugegeben, die Phasen getrennt, die ätherische Phase nacheinander mit 30 ml Wasser und gesättigter Natriumchloridlösung gewaschen, über Natriumsulfat getrocknet, filtriert, eingeengt und im Vakuum weitgehend von Lösungsmittelresten befreit. Das erhaltene rohe [8-(4-Chlorphenyl)-1,4-dioxaspiro[4.5]dec-8-yl]dimethylamin (4,18 g) wurde ohne weitere Aufreinigung zunächst für 24 Stunden mit einem Gemisch aus 10 ml konz. Salzsäure (32 Massenprozent) und 6,0 ml Wasser bei Raumtemperatur gerührt und dann drei Stunden zum Rückfluß erhitzt. Anschließend wurde die Reaktionsmischung zunächst dreimal mit je 50 ml Diethylether gewaschen, dann unter Eiskühlung durch Zugabe von konz. Ammoniaklösung (25 Massenprozent) basisch gestellt, dreimal mit je 500 ml Diethylether extrahiert, die vereinigten Diethylether-Extrakte über Natriumsulfat getrocknet, filtriert, eingeengt und im Vakuum weitgehend von Lösungsmittelresten befreit. Es wurden 3,84 g 4-(4-Chlorphenyl)-4-dimethylaminocyclohexanon als braunes Öl erhalten. 17 ml 1,0 molare Phenethylmagnesiumchloridlösung in Tetrahydrofuran wurden vorgelegt und 3,50 g 4-(4-Chlorphenyl)-4-dimethylaminocyclohexanon, gelöst in 20 ml Tetrahydrofuran, zugetropft und über Nacht bei Raumtemperatur gerührt. Zur Aufarbeitung wurden unter Eiskühlung 25 ml Ammoniumchloridlösung (20 Massenprozent) zugegeben, die Phasen getrennt, dreimal mit je 25 ml Diethylether extrahiert, die vereinigten organischen Phasen über Natriumsulfat getrocknet, filtriert, eingeengt und im Vakuum weitgehend von Lösungsmittelresten befreit. Das erhaltene Rohprodukt (4,54 g) wurde in 50 ml Diethylether aufgenommen, dreimal mit je 40 ml Salzsäure (5 Massenprozent) extrahiert und die vereinigten Extrakte zweimal mit je 40 ml Dichlormethan gewaschen. Die Dichlormethan-Extrakte wurden über Natriumsulfat getrocknet, filtriert, eingeengt und im Vakuum weitgehend von Lösungsmittelresten befreit. Es wurden 1,77 g eines gelben Harzes erhalten, das mit Diethylether/Hexan (V:V = 2:1) an Kieselgel chromatographiert wurde. Die erhaltenen 417 mg 4-(4-Chlorphenyl)-4-dimethylamino-1-phenethylcyclohexanol wurden, gelöst in 4,8 ml 2-Butanon, durch Umsetzung mit 162 μl Chlortrimethylsilan und 12μl Wasser, Rühren über Nacht mit anschließender Filtration, Diethylether-Wäsche und Vakuumtrocknung in 416 mg des korrespondierenden Hydrochlorids überführt.

**Beispiel 6**

4-(4-Bromphenyl)-4-dimethylamino-1-phenethylcyclohexanol

26,6 g 4-Bromiodbenzol wurden in 150 ml Diethylether p.a. vorgelegt und bei Raumtemperatur 47 ml 2,0 molarer Isopropylmagnesiumchloridlösung zugetropft. Nach einer weiteren Stunde wurden 18 g 8-Dimethylamino-1,4-dioxaspiro[4.5]decan-8-carbonitril, gelöst in 250 ml Diethylether, zugetropft und über Nacht gerührt. Zur Aufarbeitung wurden unter Eiskühlung 20 ml Ammoniumchloridlösung (20 Massenprozent) zugegeben, dreimal mit je 100 ml Diethylether extrahiert, die vereinigten organischen Phasen über Natriumsulfat getrocknet, filtriert, eingeengt und im Vakuum weitgehend von Lösungsmittelresten befreit. Das erhaltene Rohprodukt (21,8 g) wurde mit Diethylether an Kieselgel chromatographiert. Es wurden 7,49 g 4-(4-Bromphenyl)-4-dimethylamino-1-phenethylcyclohexanol als farblose Flüssigkeit erhalten.
7,48 g 4-(4-Bromphenyl)-4-dimethylamino-1-phenethylcyclohexanol wurden in 30 ml Diisopropylether und 10 ml Diethylether gelöst und vier Tage mit 13 ml viermolarer Salzsäure gerührt. Zur Aufarbeitung wurde die Reaktionsmischung durch Zugabe von Natronlauge (32 Massenprozent) alkalisiert, dreimal mit je 30 ml Diethylether extrahiert, die vereinigten Extrakte über Natriumsulfat getrocknet, filtriert, eingeengt und im Vakuum weitgehend von Lösungsmittelresten befreit. Die erhaltenen 5,02 g 4-(4-Bromphenyl)-4-dimethylaminocyclohexanon wurden , gelöst in 22 ml Tetrahydrofuran, unter Stickstoffatmosphäre bei Raumtemperatur zu 18 ml 1,0 molarer Phenethylmagnesiumchloridlösung in THF zugetropft und über Nacht gerührt. Zur Aufarbeitung wurden unter Eiskühlung 28 ml Ammoniumchloridlösung (20 Massenprozent) zugegeben, die Phasen getrennt, dreimal mit je 25 ml Diethylether extrahiert, die vereinigten organischen Phasen über Natriumsulfat getrocknet, filtriert, eingeengt und im Vakuum weitgehend von Lösungsmittelresten befreit. Das erhaltene Rohprodukt (5,83 g) wurde in 50 ml Diethylether aufgenommen, dreimal mit je 40 ml Salzsäure (5 Massenprozent) extrahiert und die vereinigten Extrakte zweimal mit je 40 ml Dichlormethan gewaschen. Die Dichlormethan-Extrakte wurden über Natriumsulfat getrocknet, filtriert, eingeengt und im Vakuum weitgehend von Lösungsmittelresten befreit. Es wurden 3,66 g eines hellbraunen Harzes erhalten, das mit Diethylether/Hexan (V:V = 2:1) an Kieselgel chromatographiert wurde. Die erhaltenen 1,38 g 4-(4-Bromphenyl)-4-dimethylamino-1-phenethylcyclohexanol wurden, gelöst in 20 ml 2-Butanon, durch Umsetzung mit 474 μl Chlortrimethylsilan und 34μl Wasser, Rühren über Nacht mit anschließender Filtration, Diethylether-Wäsche und Vakuumtrocknung in 1,47 mg des korrespondierenden Hydrochlorids überführt.

**Beispiel 7**

4-Dimethylamino-1-(1-methyl-1H-indol-2-yl)-4-phenylcyclohexanol

Eine Lösung von N-Methylindol (400 mg, 3,05 mmol) in trockenem THF (20 ml) wurde unter einem Argonstrom auf -5 °C gekühlt. Danach wurde *tert*-Butyllithium (3,65mmol, 2,15 ml einer 1,7 molaren Pentanlösung) so zugetropft, daß dabei eine Reaktionstemperatur von 0 °C nicht überschritten wurde. Nach beendeter Zugabe wurde die Reaktionsmischung zwei Stunden bei 0 °C gerührt. Im Anschluß wurde eine Lösung von 4-Dimethylamino-4-phenylcyclohexanon (662 mg, 3,05 mmol) in trockenem THF (5 ml) bei 0 °C zugetropft. Die Mischung wird 15 Minuten bei 0 °C und anschließend vier Stunden bei Raumtemperatur gerührt. Das Reaktionsgemisch wurde mit gesättigter Ammoniumchloridlösung (20 ml) gequencht, die organische Phase abgetrennt und die wäßrige Phase viermal mit Dichlormethan (20 ml) extrahiert. Die vereinigten organischen Phasen wurden über Natriumsulfat getrocknet, filtriert und das Lösungsmittel im Vakuum entfernt. Die Reinigung erfolgte mittels Flash-Chromatographie an Kieselgel mit Cyclohexan/Ethylacetat (V:V = 7:3). Es wurden 318 mg 4-Dimethylamino-1-(1-methyl-1H-indol-2-yl)-4-phenylcyclohexanol mit einem Schmelzpunkt von 163 - 165 °C erhalten.

**Beispiel 8**

1-Benzo[b]thiophen-2-yl-4-dimethylamino-4-phenylcyclohexanol

Eine Lösung von Benzo[b]thiophen (400 mg, 2,98 mmol) in trockenem THF (20 ml) wurde unter einem Argonstrom auf -5 °C gekühlt. Danach wurde vorsichtig *tert*-Butyllithium (3,58 mmol, 2,11 ml einer 1,7 molaren Pentanlösung) so zugetropft, daß dabei eine Reaktionstemperatur von 0 °C nicht überschritten wurde. Nach beendeter Zugabe wurde die Reaktionsmischung zwei Stunden bei 0 °C gerührt. Im Anschluß wurde eine Lösung von 4-Dimethylamino-4-phenylcyclohexanon (648 mg, 2,98 mmol) in trockenem THF (5 ml) bei 0 °C zugetropft. Die Mischung wurde 15 Minuten bei 0 °C und anschließend sechs Stunden bei Raumtemperatur gerührt. Das Reaktionsgemisch wurde mit gesättigter Ammoniumchloridlösung (20 ml) gequencht, die organische Phase abgetrennt und die wäßrige Phase viermal mit Dichlormethan (20 ml) extrahiert. Die vereinigten organischen Phasen wurden über Natriumsulfat getrocknet, filtriert und

das Lösungsmittel im Vakuum entfernt. Die Reinigung erfolgte durch Flash-Chromatographie an Kieselgel mit Cyclohexan/Ethylacetat (V:V = 1:1). Es wurden 345 mg 1-Benzo[b]thiophen-2-yl-4-dimethylamino-4-phenylcyclohexanol mit einem Schmelzpunkt von 183 - 185 °C erhalten.

**Beispiel 9**

1-Benzo[b]thiophen-3-yl-4-dimethylamino-4-phenylcyclohexanol

Eine Lösung von 3-Brom-1-benzo[b]thiophen (1,00 g, 4,69 mmol) in 30 ml trockenem Tetrahydrofuran wurde unter einem Argonstrom auf -78 °C gekühlt. Danach wurde *n*-Butyllithium (5,63 mmol, 3,52 ml einer 15 massenprozentigen. Hexanlösung) so zugetropft, daß dabei eine Reaktionstemperatur von -75 °C nicht überschritten wurde. Nach beendeter Zugabe wurde die Reaktionsmischung zwei Stunden bei - 78 °C gerührt. Im Anschluß wurde eine Lösung von 4-Dimethylamino-4-phenylcyclohexanon (1,02 g, 4,69 mmol) in trockenem Tetrahydrofuran (15 ml) bei - 78 °C zugetropft. Die Reaktionsmischung wurde zwei Stunden -78 °C gerührt und anschließend innerhalb von ca. zehn Stunden auf Raumtemperatur aufgetaut. Das Reaktionsgemisch wurde mit gesättigter Ammoniumchloridlösung (30 ml) gequencht, die organische Phase abgetrennt und die wäßrige Phase viermal mit Dichlormethan (25 ml) extrahiert. Die vereinigten organischen Phasen wurden über Natriumsulfat getrocknet, filtriert und das Lösungsmittel im Vakuum entfernt. Die Reinigung erfolgt mittels Flash-Chromatographie an Kieselgel mit Cyclohexan/Ethylacetat (V:V = 7:3). Es wurden 445 mg 1-Benzo[b]thiophen-3-yl-4-dimethylamino-4-phenylcyclohexanol mit einem Schmelzpunkt von 176 - 178 °C erhalten.

**Beispiel 10**

1-(1-Benzolsulfonyl-1H-indol-2-yl)-4-dimethylamino-4-phenylcyclohexanol

Eine Lösung von 1-Benzolsulfonyl-1*H*-indol (600 mg, 2,33 mmol) in trockenem THF (30 ml) wurde unter einem Argonstrom auf -5 °C gekühlt. Danach wurde *n*-Butyllithium (2,79 mmol, 1,75 ml einer 1,6 molaren Hexanlösung) so zugetropft, daß dabei eine Reaktionstemperatur von 0 °C nicht überschritten wurde. Nach beendeter Zugabe wurde die Reaktionsmischung zwei Stunden bei 0 °C gerührt. Im Anschluß wurde eine Lösung von 4-Dimethylamino-4-phenylcyclohexanon (493 mg, 2,33 mmol) in trockenem THF (8 ml) bei 0 °C zugetropft. Die Mischung wurde eine Stunde bei 0 °C und anschließend drei Tage bei Raumtemperatur gerührt. Das Reaktionsgemisch wurde mit gesättigter Ammoniumchloridlösung (20 ml) gequencht, die organische Phase abgetrennt und die wäßrige Phase viermal mit Dichlormethan (20 ml) extrahiert. Die vereinigten organischen Phasen wurden über Natriumsulfat getrocknet, filtriert und das Lösungsmittel im Vakuum entfernt. Die Reinigung erfolgte mittels Flash-Chromatographie an Kieselgel mit Cyclohexan/Ethylacetat (V:V = 1:1). Es wurden 232 mg 1-(1-Benzolsulfonyl-1 H-indol-2-yl)-4-dimethylamino-4-phenylcyclohexanol mit einem Schmelzpunkt von 173 - 176 °C erhalten.

**Beispiel 11**

1-Benzofuran-2-yl-4-dimethylamino-4-phenylcyclohexanol

Eine Lösung von Benzo[*b*]furan (1,50 g, 12,7 mmol) in trockenem THF (50 ml) wurde unter einem Argonstrom auf -8 °C gekühlt. Danach wurde *tert*-Butyllithium (15,2 mmol, 10,2 ml einer 1,5 molaren Pentanlösung) so zugetropft, daß dabei eine Reaktionstemperatur von -5 °C nicht überschritten wurde. Nach beendeter Zugabe wurde die Reaktionsmischung zweieinhalb Stunden bei -5 °C gerührt. Im Anschluß wurde eine Lösung von 4-Dimethylamino-4-phenylcyclohexanon (2,76 g, 12,7 mmol) in trockenem THF (15 ml) bei 0 °C zugetropft. Die Mischung wurde eine Stunde bei 0 °C und anschließend vier Tage bei Raumtemperatur gerührt. Das Reaktionsgemisch wurde mit gesättigter Ammoniumchloridlösung (30 ml) gequencht, die organische Phase abgetrennt und die wäßrige Phase viermal mit Dichlormethan (30 ml) extrahiert. Die vereinigten organischen Phasen wurden über Natriumsulfat getrocknet, filtriert und das Lösungsmittel im Vakuum entfernt. Die Reinigung erfolgte mittels Flash-Chromatographie an Kieselgel mit Cyclohexan/Ethylacetat (V:V = 7:3). Es wurden 1,02 g 1-Benzofuran-2-yl-4-dimethylamino-4-phenylcyclohexanol mit einem Schmelzpunkt von 134 - 137 °C erhalten.

**Beispiel 12**

1-Benzothiazol-2-yl-4-dimethylamino-4-phenylcyclohexanol

Eine Lösung von Benzothiazol (622 mg, 4,60 mmol) in trockenem THF (30 ml) wurde unter einem Argonstrom

auf -85 °C gekühlt. Danach wurde *n*-Butyllithium (5,52 mmol, 3,45 ml einer 1,6 molaren Hexanlösung) so zugetropft, daß dabei eine Reaktionstemperatur von -78 °C nicht überschritten wurde. Nach beendeter Zugabe wurde die Reaktionsmischung neunzig Minuten bei -80 °C gerührt. Im Anschluß wurde eine Lösung von 4-Dimethylamino-4-phenylcyclohexanon (1,00 g, 4,61 mmol) in trockenem THF (8 ml) bei -80 °C zugetropft. Die Mischung wurde 30 Minuten bei -80 °C und anschließend drei Tage bei Raumtemperatur gerührt. Das Reaktionsgemisch wurde mit gesättigter Ammoniumchloridlösung (30 ml) gequencht, die organische Phase abgetrennt und die wäßrige Phase viermal mit Dichlormethan (30 ml) extrahiert. Die vereinigten organischen Phasen wurden über Natriumsulfat getrocknet, filtriert und das Lösungsmittel im Vakuum entfernt. Die Reinigung erfolgte mittels Flash-Chromatographie an Kieselgel mit Cyclohexan/Ethylacetat (V:V = 7:3). Es wurden 746 mg 1-Benzothiazol-2-yl-4-dimethylamino-4-phenylcyclohexanol mit einem Schmelzpunkt von 155 - 157 °C erhalten.

**Patentansprüche**

**1.** Substituierte 4-Aminocyclohexanole der allgemeinen Formel I,

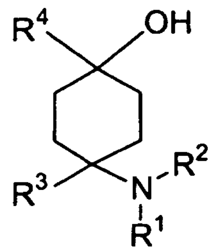

I

, worin
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,
mit $R^5$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus Aryl oder Heteroaryl, jeweils unsubstituiert oder einfach oder mehrfach substituiert;
$R^4$ ausgewählt ist aus $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$
mit Y = O, S oder $H_2$,
mit $R^6$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
mit $R^7$ ausgewählt aus
H; $C_{3-8}$-Cycloalkyl, Aryl oder Heteroaryl, jeweils unsubstituiert oder einfach oder mehrfach substituiert,
mit $R^8$ ausgewählt aus
Aryl oder Heteroaryl, jeweils unsubstituiert oder einfach oder mehrfach substituiert,
mit L ausgewählt aus
$-C(O)-NH-$, $-NH-C(O)-$, $-C(O)-O-$, $-O-C(O)-$, $-O-$, $-S-$ oder $-S(O)_2-$
mit $R^9$ ausgewählt aus
Aryl oder Heteroaryl, jeweils unsubstituiert oder einfach oder mehrfach substituiert,

mit der Maßgabe, daß,
wenn $R^3$ = Phenyl, substituiert oder unsubstituiert, ist und $R^4$ = Phenyl oder $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$
mit Y = $H_2$,
$R^6$ = H, $C_{1-5}$-Alkyl, gesättigt oder ungesättigt, verzweigt oder unverzweigt, einfach oder mehrfach substituiert oder unsubstituiert
und/oder
$R^7$ = H, $C_{3-8}$-Cycloalkyl oder Phenyl, jeweils substituiert oder unsubstituiert, bedeuten,
$R^1$ und $R^2$ nicht beide unabhängig voneinander $C_{1-5}$-Alkyl sind,
wenn $R^3$ = Thiophenyl, substituiert oder unsubstituiert, und $R^4$ = $-CH_2-CH_2$-Phenyl
die Reste $R^1$ und $R^2$ nicht zusammen einen Ring bilden und $(CH_2)_5$ bedeuten,
wobei "Aryl oder Heteroaryl einfach oder mehrfach substituiert" für "Aryl oder Heteroaryl einfach oder mehrfach substituiert mit $R^{82}$, $OR^{82}$, Halogen, $CF_3$, CN, $NO_2$, $NR^{83}R^{84}$, $C_{1-6}$-Alkyl, $C_{1-6}$-Alkoxy, $C_{3-8}$-Cycloalkoxy, $C_{3-8}$-Cycloalkyl oder $C_{2-6}$-Alkylen" steht
wobei
$R^{82}$ für H, $C_{1-10}$-Alkyl-, Aryl, Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, steht;
$R^{83}$ und $R^{84}$ gleich oder verschieden, H, $C_{1-10}$-Alkyl, Aryl, Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste $R^{83}$ und $R^{84}$ zusammen $CH_2CH_2OCH_2CH_2$, $CH_2CH_2NR^{85}CH_2CH_2$ oder $(CH_2)_{3-6}$ bedeuten, und
der Rest $R^{85}$ für H, $C_{1-10}$-Alkyl, Aryl, Heteroaryl oder für einen über $C_{1-3}$-Alkyl, gesättigt oder ungesättigt, oder eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen;
"Cycloalkyl, einfach oder mehrfach substituiert" für Cycloalkyl, einfach oder mehrfach substituiert mit F, Cl, Br, I, $NH_2$, SH, OH, $OC_{1-3}$-Alkyl oder $C_{1-3}$-Alkyl" steht
und
"Alkyl, einfach oder mehrfach substituiert" für "Alkyl, einfach oder mehrfach substituiert mit F, Cl, Br, I, $NH_2$, SH oder OH" steht,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

**2.** Substituierte 4-Aminocyclohexanole gemäß Anspruch 1, worin die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten.

**3.** Substituierte 4-Aminocyclohexanole gemäß Anspruch 1, worin $R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen.

**4.** Substituierte 4-Aminocyclohexanole gemäß Anspruch 1, worin $R^3$ ausgewählt ist aus Heteroaryl, jeweils unsubstituiert oder einfach oder mehrfach substituiert.

**5.** Substituierte 4-Aminocyclohexanole gemäß Anspruch 1, worin $R^3$ ausgewählt ist aus Aryl, unsubstituiert oder einfach oder mehrfach substituiert.

**6.** Substituierte 4-Aminocyclohexanole gemäß Anspruch 1, worin die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten und $R^3$ ausgewählt ist aus Aryl, jeweils unsubstituiert oder einfach oder mehrfach substituiert.

**7.** Substituierte 4-Aminocyclohexanole gemäß Anspruch 1, worin $R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder

mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, und $R^3$ ausgewählt ist aus Heteroaryl, unsubstituiert oder einfach oder mehrfach substituiert.

**8.** Substituierte 4-Aminocyclohexanole gemäß Anspruch 1, worin $R^7$ ausgewählt ist aus Heteroaryl, jeweils unsubstituiert oder einfach oder mehrfach substituiert,

**9.** Substituierte 4-Aminocyclohexanole gemäß Anspruch 1, worin $R^4$ ausgewählt ist aus $-CHR^6R^7$, $-CHR^6- CH_2R^7$, $-CHR^6-CH_2-CH_2R^7$ oder $-CHR^6-CH_2-CH_2-CH_2R^7$
mit $R^6$ ausgewählt aus C(O)O-$C_{1-6}$-Alkyl, gesättigt oder ungesättigt, verzweigt oder unverzweigt, einfach oder mehrfach substituiert oder unsubstituiert.

**10.** Substituierte 4-Aminocyclohexanole gemäß Anspruch 1, worin $R^4$ ausgewählt ist aus $-C(Y)R^7$, $-C(Y)-CH_2R^7$, $-C(Y)-CH_2-CH_2R^7$ oder $-C(Y)-CH_2-CH_2-CH_2R^7$ mit Y = O oder S.

**11.** Substituierte 4-Aminocyclohexanole gemäß einem der Ansprüche 1, 4, 5, 8, 9 oder 10, **dadurch gekennzeichnet, daß**
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,
mit $R^5$ ausgewählt aus H; $C_{1-8}$-Alkyl, gesättigt oder ungesättigt, verzweigt oder unverzweigt, einfach oder mehrfach substituiert oder unsubstituiert,
vorzugsweise $R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

**12.** Substituierte 4-Aminocyclohexanole gemäß einem der Ansprüche 3 oder 7,
**dadurch gekennzeichnet, daß**
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
insbesondere
$R^1$ und $R^2$ unabhängig voneinander ausgewählt sind aus Methyl oder Ethyl.

**13.** Substituierte 4-Aminocyclohexanole gemäß einem der Ansprüche 2 oder 6,
**dadurch gekennzeichnet, daß**
$R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,
mit $R^5$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ zusammen einen Ring bilden und $(CH_2)_{4-5}$ bedeuten,
insbesondere
$R^1$ und $R^2$ zusammen einen Ring bilden und $(CH_2)_5$ bedeuten.

**14.** Substituierte 4-Aminocyclohexanole gemäß einem der Ansprüche 1, 2, 3, 8, 9 oder 10, **dadurch gekennzeichnet, daß**
$R^3$ ausgewählt ist aus Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;

vorzugsweise
$R^3$ ausgewählt ist aus Phenyl, Thiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Pyridyl, Furyl oder Thiophenyl, jeweils unsubstituiert oder einfach oder mehrfach substituiert.

**15.** Substituierte 4-Aminocyclohexanole gemäß einem der Ansprüche 4 oder 7,
**dadurch gekennzeichnet, daß**
$R^3$ ausgewählt ist aus Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
vorzugsweise
$R^3$ ausgewählt ist aus Thiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Pyridyl, Furyl oder Thiophenyl, jeweils unsubstituiert oder einfach oder mehrfach substituiert.

**16.** Substituierte 4-Aminocyclohexanole gemäß einem der Ansprüche 5 oder 6,
**dadurch gekennzeichnet, daß**
$R^3$ ausgewählt ist aus Phenyl, Naphthyl, Anthracenyl;
vorzugsweise
$R^3$ ausgewählt ist aus Phenyl oder Naphthyl, jeweils unsubstituiert oder einfach oder mehrfach substituiert;
insbesondere
$R^3$ ausgewählt ist aus Phenyl, unsubstituiert oder einfach oder mehrfach substituiert.

**17.** Substituierte 4-Aminocyclohexanole gemäß einem der Ansprüche 1 bis 7,
**dadurch gekennzeichnet, daß**
$R^4$ ausgewählt ist aus $C_{3-8}$-Cycloalkyl, Aryl oder Heteroaryl, jeweils unsubstituiert oder einfach oder mehrfach substituiert; oder $-R^8-L-R^9$
vorzugsweise
$R^4$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert; oder $-R^8-L-R^9$
insbesondere
$R^4$ ausgewählt ist aus Cyclopentyl, Cyclohexyl, Cycloheptyl, Cyclooctyl, Anthracenyl, Indolyl, Naphthyl, Benzofuranyl, Benzothiazolyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert; oder $-R^8-L-R^9$.

**18.** Substituierte 4-Aminocyclohexanole gemäß Anspruch 17, **dadurch gekennzeichnet, daß**
$R^8$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
L ausgewählt aus
-C(O)-NH-, -NH-C(O)-, -C(O)-O-, -O-C(O)-, -O-, -S- oder $-S(O)_2-$,
und/oder $R^9$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
vorzugsweise

$R^8$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert,
L ausgewählt aus
-C(O)-NH-, -NH-C(O)-, -C(O)-O-, -O-C(O)- oder $-S(O)_2-$,
und/oder $R^9$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert
insbesondere
$R^8$ ausgewählt ist aus
Indolyl, unubstituiert,
L ausgewählt aus
$-S(O)_2-$
und $R^9$ ausgewählt ist aus
Phenyl unsubstituiert.

**19.** Substituierte 4-Aminocyclohexanole gemäß einem der Ansprüche 1 bis 7,
**dadurch gekennzeichnet, daß**
$R^4$ ausgewählt ist aus $-CHR^6R^7$, $-CHR^6-$ $CH_2R^7$, $-CHR^6-CH_2-CH_2R^7$, - $CHR^6-CH_2-CH_2-CH_2R^7$, $-C(Y)R^7$, -C (Y)-$CH_2R^7$, -C(Y)-$CH_2$-$CH_2R^7$ oder - C(Y)-$CH_2$-$CH_2$-$CH_2R^7$
mit Y = O, S oder $H_2$,
vorzugsweise
$R^4$ ausgewählt ist aus $-CHR^6R^7$, $-CHR^6-$ $CH_2R^7$, $-CHR^6-CH_2-H_2R^7$, - $C(Y)R^7$, -C(Y)-$CH_2R^7$ oder -C(Y)-$CH_2$-$CH_2R^7$
mit Y = O oder S,
insbesondere
$R^4$ ausgewählt ist aus $-CHR^6R^7$, $-CHR^6-$ $CH_2R^7$, $-C(Y)R^7$ oder -C(Y)-$CH_2R^7$
mit Y = O.

**20.** Substituierte 4-Aminocyclohexanole gemäß Anspruch 19, **dadurch gekennzeichnet, daß**
$R^6$ ausgewählt ist aus
H, $C_{1-4}$-Alkyl, gesättigt oder ungesättigt, verzweigt oder unverzweigt, einfach oder mehrfach substituiert oder unsubstituiert; oder C(O)O-$C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert;
insbesondere
H, $CH_3$ und $C_2H_5$.

**21.** Substituierte 4-Aminocyclohexanole gemäß Anspruch 19, **dadurch gekennzeichnet, daß**
$R^7$ ausgewählt ist aus $C_{3-8}$-Cycloalkyl, Aryl oder Heteroaryl, jeweils unsubstituiert oder einfach oder mehrfach substituiert;
vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**22.** Substituierte 4-Aminocyclohexanole gemäß Anspruch 8, **dadurch gekennzeichnet, daß**
$R^4$ ausgewählt ist aus Heteroaryl, jeweils unsubstituiert oder einfach oder mehrfach substituiert; oder $-CHR^6R^7$, $-CHR^6-$ $CH_2R^7$, $-CHR^6-CH_2-CH_2R^7$, $-CHR^6-CH_2-CH_2-CH_2R^7$, $-C(Y)R^7$, -C(Y)-$CH_2R^7$, -C(Y)-$CH_2$-$CH_2R^7$ oder - C (Y)-$CH_2$-$CH_2$-$CH_2R^7$; oder $-R^8-L-R^9$

mit Y = $H_2$,
vorzugsweise
$R^4$ ausgewählt ist aus Indolyl, Benzofuranyl, Benzothiophenyl, Indanyl, Benzodioxanyl, Benzodioxolanyl, Acenaphthyl, Carbazolyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert; oder $-CHR^6R^7$, $-CHR^6-CH_2R^7$, $-CHR^6-CH_2-CH_2R^7$, $-C(Y)R^7$, $-C(Y)-CH_2R^7$ oder $-C(Y)-CH_2-CH_2R^7$
mit Y = $H_2$,
insbesondere
$R^4$ ausgewählt ist aus Indolyl, Benzofuranyl, Benzothiophenyl, Benzothiazolyl, Indanyl, Benzodioxanyl, Benzodioxolanyl, Acenaphthyl, Carbazolyl, Thiophenyl, Furyl, Pyridyl, Pyrrolyl, Pyrazinyl oder Pyrimidyl, jeweils unsubstituiert oder einfach oder mehrfach substituiert; oder $-CHR^6R^7$, $-CHR^6-CH_2R^7$, $-C(Y)R^7$ oder $-C(Y)-CH_2R^7$,
mit Y = $H_2$.

**23.** Substituierte 4-Aminocyclohexanole gemäß Anspruch 22, **dadurch gekennzeichnet, daß**
$R^6$ ausgewählt ist aus
H, $C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert;
insbesondere
H, $CH_3$ und $C_2H_5$
und/oder
$R^7$ ausgewählt ist aus Indolyl, Benzofuranyl, Benzothiophenyl, Indanyl, Benzodioxanyl, Benzodioxolanyl, Acenaphthyl, Carbazolyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert;
vorzugsweise
$R^7$ ausgewählt ist aus Indolyl, Benzofuranyl, Benzothiophenyl, Indanyl, Benzodioxanyl, Benzodioxolanyl, Acenaphthyl, Carbazolyl, Thiophenyl, Furyl, Pyridyl, Pyrrolyl, Pyrazinyl oder Pyrimidyl, jeweils unsubstituiert oder einfach oder mehrfach substituiert;
insbesondere
$R^7$ ausgewählt ist aus Thiophenyl, Furyl, Pyridyl, Pyrrolyl, Pyrazinyl oder Pyrimidyl, jeweils unsubstituiert oder einfach oder mehrfach substituiert.

**24.** Substituierte 4-Aminocyclohexanole gemäß Anspruch 22, **dadurch gekennzeichnet, daß**
$R^8$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
L ausgewählt aus
-C(O)-NH-, -NH-C(O)-, -C(O)-O-, -O-C(O)-, -O-, -S- oder $-S(O)_2-$,
und/oder $R^9$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
vorzugsweise
$R^8$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert,
L ausgewählt aus

-C(O)-NH-, -NH-C(O)-, -C(O)-O-, -O-C(O)- oder $-S(O)_2-$,
und/oder $R^9$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert
insbesondere
$R^8$ ausgewählt ist aus
Indolyl, unubstituiert,
L ausgewählt aus

$-S(O)_2-$

und $R^9$ ausgewählt ist aus
Phenyl unsubstituiert.

**25.** Substituierte 4-Aminocyclohexanole gemäß Anspruch 9, **dadurch gekennzeichnet, daß**
$R^4$ ausgewählt ist aus $-CHR^6R^7$, $-CHR^6-CH_2R^7$ oder $-CHR^6-CH_2-CH_2R^7$
vorzugsweise
$R^4$ ausgewählt ist aus $-CHR^6R^7$ oder $-CHR^6-CH_2R^7$,
insbesondere
$R^4$ ausgewählt ist aus $-CHR^6R^7$.

**26.** Substituierte 4-Aminocyclohexanole gemäß Anspruch 25, **dadurch gekennzeichnet, daß**
$R^6$ ausgewählt ist aus
$C(O)O-C_{1-4}$-Alkyl, gesättigt oder ungesättigt, verzweigt oder unverzweigt, einfach oder mehrfach substituiert oder unsubstituiert;
vorzugsweise
$C(O)O-C_{1-3}$-Alkyl, gesättigt oder ungesättigt, verzweigt oder unverzweigt, einfach oder mehrfach substituiert oder unsubstituiert;
insbesondere
$C(O)O-CH_3$ und $C(O)O-C_2H_5$
und/oder
$R^7$ ausgewählt ist aus $C_{3-8}$-Cycloalkyl, Aryl oder Heteroaryl, jeweils unsubstituiert oder einfach oder mehrfach substituiert;
vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**27.** Substituierte 4-Aminocyclohexanole gemäß Anspruch 10, **dadurch gekennzeichnet, daß**
$R^4$ ausgewählt ist aus $-C(Y)R^7$, $-C(Y)-CH_2R^7$, $-C(Y)-CH_2-CH_2R^7$ oder $-C(Y)-CH_2-CH_2-CH_2R^7$
mit Y = O
vorzugsweise
$R^4$ ausgewählt ist aus $-C(Y)R^7$, $-C(Y)-CH_2R^7$ oder $-C(Y)-CH_2-CH_2R^7$,
mit Y = O
insbesondere
$R^4$ ausgewählt ist aus $-C(Y)R^7$ oder $-C(Y)-CH_2R^7$,
mit Y = O.

**28.** Substituierte 4-Aminocyclohexanole gemäß Anspruch 27, **dadurch gekennzeichnet, daß**
$R^7$ ausgewählt ist aus $C_{3-8}$-Cycloalkyl, Aryl oder Heteroaryl, jeweils unsubstituiert oder einfach oder mehrfach substituiert;

vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**29.** Substituierte 4-Aminocyclohexanole gemäß einem der Ansprüche 1 bis 28,
**dadurch gekennzeichnet, daß** sie ausgewählt sind aus der folgenden Gruppe:

- 4-Dimethylamino-1-(1-methyl-1H-indol-2-yl)-4-phenylcyclohexanol
- 1-Benzo[b]thiophen-2-yl-4-dimethylamino-4-phenylcyclohexanol
- 1-Benzo[b]thiophen-3-yl-4-dimethylamino-4-phenylcyclohexanol
- 1-(1-Benzolsulfonyl-1H-indol-2-yl)-4-dimethylamino-4-phenylcyclohexanol
- 1-Benzofuran-2-yl-4-dimethylamino-4-phenylcyclohexanol
- 1-Benzothiazol-2-yl-4-dimethylamino-4-phenylcyclohexanol gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

**30.** Arzneimittel enthaltend wenigstens ein substituiertes 4-Aminocyclohexanol gemäß einem der Ansprüche 1 bis 29 gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; sowie gegebenenfalls geeignete Zusatz- und/oder Hilfsstoffe und/oder gegebenenfalls weitere Wirkstoffe.

**31.** Arzneimittel gemäß Anspruch 30, **dadurch gekennzeichnet, daß** das Arzneimittel neben wenigstens einem substituierten 4-Aminocyclohexanol noch ein Opioid, vorzugsweise ein starkes Opioid, insbesondere Morphin, oder ein Anesthetikum, vorzugsweise Hexobarbital oder Halothan, enthält.

**32.** Verwendung eines substituierten 4-Aminocyclohexanols gemäß einem der Ansprüche 1 bis 29 gegebenenfalls in Form seines Racemats, der reinen Stereoisomeren, insbesondere Enantiomeren oder Diastereomeren, oder in Form von Mischungen der Stereoisomeren, insbesondere der Enantiomeren der Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; zur Herstellung eines Arzneimittels zur Behandlung von Schmerz, insbesondere von akutem, viszeralem, neuropathischem oder chronischem Schmerz; von Migräne oder lokomotorischen Störungen; als Antikonvulsivum oder Muskelrelaxanz.

**33.** Verwendung eines substituierten 4-Aminocyclohexanols der allgemeinen Formel I,

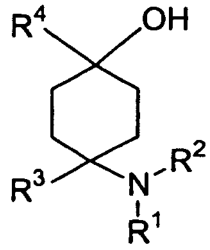

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_{2C}H_2OCH_2CH_2$, $CH_2CH_2NR^5CH_2CH_2$ oder $(CH_2)_{3-6}$ bedeuten,

mit $R^5$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus Aryl oder Heteroaryl, jeweils unsubstituiert oder einfach oder mehrfach substituiert;

$R^4$ ausgewählt ist aus $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$

mit Y = O, S oder $H_2$,

mit $R^6$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit $R^7$ ausgewählt aus

H; $C_{3-8}$-Cycloalkyl, Aryl oder Heteroaryl, jeweils unsubstituiert oder einfach oder mehrfach substituiert,

mit $R^8$ ausgewählt aus

Aryl oder Heteroaryl, jeweils unsubstituiert oder einfach oder mehrfach substituiert,

mit L ausgewählt aus

-C(O)-NH-, -NH-C(O)-, -C(O)-O-, -O-C(O)-, -O-, -S- oder $-S(O)_2-$

mit $R^9$ ausgewählt aus

Aryl oder Heteroaryl, jeweils unsubstituiert oder einfach oder mehrfach substituiert,

wobei "Aryl oder Heteroaryl einfach oder mehrfach substituiert" für "Aryl oder Heteroaryl, einfach oder mehrfach substituiert mit $R^{82}$, $OR^{82}$, Halogen, $CF_3$, CN, $NO_2$, $NR^{83}R^{84}$, $C_{1-6}$-Alkyl, $C_{1-6}$-Alkoxy, $C_{3-8}$-Cycloalkoxy, $C_{3-8}$-Cycloalkyl oder $C_{2-6}$-Alkylen" steht

wobei

$R^{82}$ für H, $C_{1-10}$-Alkyl, Aryl Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, steht;

$R^{83}$ und $R^{84}$ gleich oder verschieden, H, $C_{1-10}$-Alkyl, Aryl, Heteroaryl oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{83}$ und $R^{84}$ zusammen $CH_2CH_2OCH_2CH_2$, $CH_2CH_2NR^{85}CH_2CH_2$ oder $(CH_2)_{3-6}$ bedeuten, und der Rest $R^{85}$ für H, $C_{1-10}$-Alkyl, Aryl, oder Heteroaryl oder für einen über $C_{1-3}$-Alkyl, gesättigt oder ungesättigt, oder eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen;

"Cycloalkyl, einfach oder mehrfach substituiert" für "Cycloalkyl, einfach oder mehrfach substituiert mit F, Cl, Br, I, $NH_2$, SH, OH, $OC_{1-3}$-Alkyl oder $C_{1-3}$-Alkyl" steht

und

"Alkyl, einfach oder mehrfach substituiert" für "Alkyl, einfach oder mehrfach substituiert mit F, Cl, Br, I, $NH_2$, SH oder OH" steht,

gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Schwerhörigkeit, mangelnder Darmmotilität, gestörter Nahrungsaufnahme, Anorexie, Fettsucht, Diarrhoe, Kachexie, Harninkontinenz bzw. als Antitussivum oder Anesthetikum bzw. zur Coadministration bei Behandlung mit einem opioiden Analgetikum oder mit einem Anesthetikum, zur Diurese oder Antinatriurese und/oder Anxiolyse.

**34.** Verfahren zur Herstellung eines substituierten 4-Aminocyclohexanols gemäß einem der Ansprüche 1 bis 29 mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

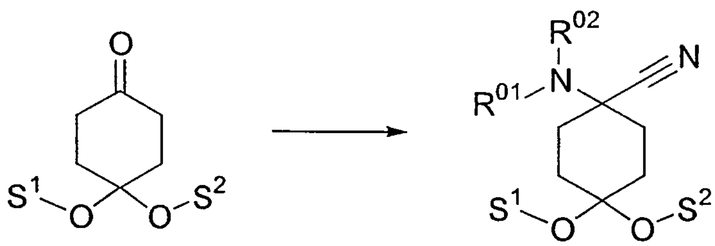

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit metallorganischen Reagenzien, bevorzugt Grignard- oder Organolithiumreagenzien, der Formel Metall-$R^3$ umgesetzt, so daß eine Verbindung gemäß Formel IVa entsteht;

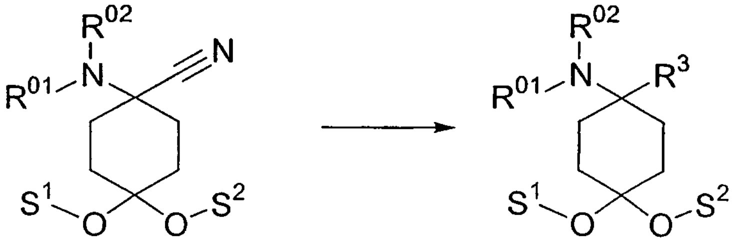

III IVa

gegebenenfalls wird anschließend in beliebiger Reihenfolge und gegebenfalls wiederholt acyliert, alkyliert oder

sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa gemäß Formel III werden die Schutzgruppen $S^1$ und $S^2$ abgespalten, so daß ein 4-substituiertes 4-Aminocyclohexanonderivat gemäß Formel IV entsteht;

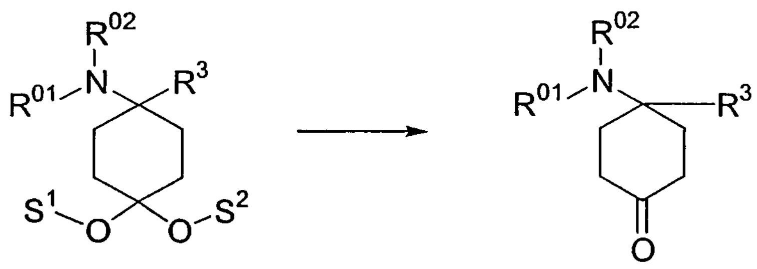

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IV mit metallorganischen Reagenzien, bevorzugt Grignard- oder Organolithiumreagenzien, der Formel Metall-$R^{04}$ umgesetzt, so daß eine Verbindung gemäß Formel V entsteht;

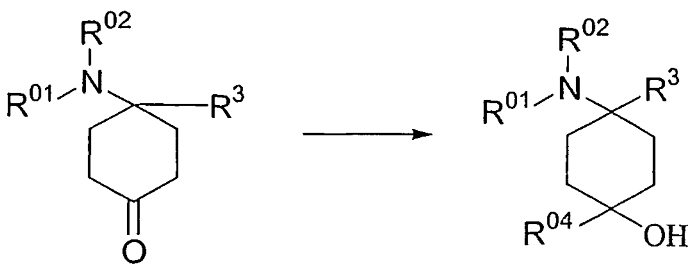

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$ und $R^4$ die in Anspruch 1 angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
oder die Reste $R^{01}$ und $R^{02}$ zusammen einen Ring bilden und $CH_2CH_2OCH_2CH_2$, $CH_2CH_2NR^{05}CH_2CH_2$ oder

$(CH_2)_{3-6}$ bedeuten,
mit $R^{05}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$
mit Y = O, S oder $H_2$,
mit $R^6$ ausgewählt aus
H, $C_{1-7}$-Alkyl, gesättigt oder ungesättigt, verzweigt oder unverzweigt, einfach oder mehrfach substituiert oder unsubstituiert;
und mit $R^7$ ausgewählt aus
H; $C_{3-8}$-Cycloalkyl, Aryl oder Heteroaryl, jeweils unsubstituiert oder einfach oder mehrfach substituiert,
mit $R^8$ ausgewählt aus
Aryl oder Heteroaryl, jeweils unsubstituiert oder einfach oder mehrfach substituiert,
mit L ausgewählt aus
-C(O)-NH-, -NH-C(O)-, -C(O)-O-, -O-C(O)-, -O-, -S- oder $-S(O)_2-$
mit $R^9$ ausgewählt aus
Aryl oder Heteroaryl, jeweils unsubstituiert oder einfach oder mehrfach substituiert,
und $S^1$ und $S^2$ unabhängig voneinander ausgewählt sind aus Schutzgruppen oder zusammen eine Schutzgruppe bedeuten, vorzugsweise Monoacetal.

**35.** Verfahren zur Herstellung eines substituierten 4-Aminocyclohexanols gemäß Anspruch 34, **dadurch gekennzeichnet, daß** die Schutzgruppen am H bei $R^{01}$, $R^{02}$, $R^{04}$ und/oder $R^{05}$ ausgewählt sind aus Alkyl, Benzyl oder Carbamaten, beispielsweise FMOC, Z oder Boc.

## Claims

**1.** Substituted 4-aminocyclohexanols of general formula I,

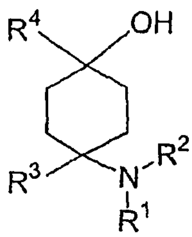

I

wherein
$R^1$ and $R^2$ independently of one another are selected from H; respectively saturated or unsaturated, branched or unbranched, singly or multiply substituted $C_{1-8}$-alkyl or $C_{3-8}$-cycloalkyl; respectively singly or multiply substituted or unsubstituted aryl or heteroaryl; or respectively singly or multiply substituted or unsubstituted $C_{1-3}$-alkylene-bound aryl, $C_{3-8}$-cycloalkyl or heteroaryl; wherein $R^1$ and $R^2$ may not both be H,
or the radicals $R^1$ and $R^2$ together form a ring and represent $CH_2CH_2OCH_2CH_2$, $CH_2CH_2NR^5CH_2CH_2$ or $(-CH_2)_{3-6}$, where $R^5$ is selected from H; respectively saturated or unsaturated, branched or unbranched, singly or multiply substituted or unsubstituted $C_{1-8}$-alkyl or $C_{3-8}$-cycloalkyl; respectively singly or multiply substituted or unsubstituted aryl or heteroaryl; or respectively singly or multiply substituted or unsubstituted $C_{1-3}$-alkylene-bound aryl, $C_{3-8}$-cycloalkyl or heteroaryl;
$R^3$ is selected from respectively unsubstituted or singly or multiply substituted aryl or heteroaryl;
$R^4$ is selected from respectively unsubstituted or singly or multiply substituted $C_{3-8}$-cycloalkyl, aryl or heteroaryl;

$-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$; or $-R^8-L-R^9$
where Y = O, S or $H_2$,
where $R^6$ is selected from
H, saturated or unsaturated, branched or unbranched, singly or multiply substituted or unsubstituted $C_{1-7}$-alkyl; or saturated or unsaturated, branched or unbranched, singly or multiply substituted or unsubstituted C(O)O-$C_{1-6}$-alkyl;
where $R^7$ is selected from
H; respectively unsubstituted or singly or multiply substituted $C_{3-8}$-cycloalkyl, aryl or heteroaryl,
where $R^8$ is selected from
respectively unsubstituted or singly or multiply substituted aryl or heteroaryl,
where L is selected from
-C(O)-NH-, -NH-C(O)-, -C(O)-O-, -O-C(O)-, -O-, -S- or $-S(O)_2-$
where $R^9$ is selected from
respectively unsubstituted or singly or multiply substituted aryl or heteroaryl,
provided that,

- if $R^3$ = substituted or unsubstituted phenyl, and $R^4$ = phenyl or $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$
where Y = $H_2$
$R^6$ = H, saturated or unsaturated, branched or unbranched, singly or multiply substituted or unsubstituted $C_{1-5}$-alkyl,
and/or
$R^7$ = H, respectively substituted or unsubstituted $C_{3-8}$-cycloalkyl or phenyl,
$R^1$ and $R^2$ independently of one another are not both $C_{1-5}$-alkyl,
- if $R^3$ = substituted or unsubstituted thiophenyl, and $R^4$ = $-CH_2-CH_2$-phenyl
the radicals $R^1$ and $R^2$ do not together form a ring and represent $(CH_2)_5$,

wherein "singly or multiply substituted aryl or heteroaryl" means "aryl or heteroaryl singly or multiply substituted with $R^{82}$, $OR^{82}$, halogen, $CF_3$, CN, $NO_2$, $NR^{83}R^{84}$, $C_{1-6}$ alkyl, $C_{1-6}$ alkoxy, $C_{3-8}$ cycloalkoxy, $C_{3-8}$ cycloalkyl or $C_{2-6}$ alkylene",
wherein
$R^{82}$ represents H, $C_{1-10}$ alkyl, aryl, heteroaryl or an aryl or heteroaryl radical which is bound via $C_{1-3}$ alkyl, saturated or unsaturated, or a $C_{1-3}$ alkylene group, wherein these aryl and heteroaryl radicals may not themselves be substituted with aryl or heteroaryl radicals;
$R^{83}$ and $R^{84}$ are the same or different and represent H, $C_{1-10}$ alkyl, aryl, heteroaryl or an aryl or heteroaryl radical which is bound via $C_{1-3}$ alkyl, saturated or unsaturated, or a $C_{1-3}$ alkylene group, wherein these aryl and heteroaryl radicals may not themselves be substituted with aryl or heteroaryl radicals,
or the radicals $R^{83}$ and $R^{84}$ together represent $CH_2CH_2OCH_2CH_2$, $CH_2CH_2NR^{85}CH_2CH_2$ or $(CH_2)_{3-6}$, and
the radical $R^{85}$ represents H, $C_{1-10}$ alkyl, aryl, heteroaryl or an aryl or heteroaryl radical which is bound via $C_{1-3}$ alkyl, saturated or unsaturated, or a $C_{1-3}$ alkylene group, wherein these aryl and heteroaryl radicals may not themselves be substituted with aryl or heteroaryl radicals;
"singly or multiply substituted cycloalkyl" means "cycloalkyl singly or multiply substituted with F, Cl, Br, I, $NH_2$, SH, OH, $OC_{1-3}$ alkyl or $C_{1-3}$ alkyl",
and
"singly or multiply substituted alkyl" means "alkyl singly or multiply substituted with F, Cl, Br, I, $NH_2$, SH or OH",
optionally in the form of their racemates, their pure stereoisomers, in particular enantiomers or diastereomers, or in the form of mixtures of stereoisomers, in particular the enantiomers or diastereomers, in any mixing ratio; in the prepared form or in the form of their acids or bases or in the form of their salts, in particular the physiologically acceptable salts or in the form of their solvates, in particular the hydrates.

**2.** Substituted 4-aminocyclohexanols according to claim 1, wherein the radicals $R^1$ and $R^2$ together form a ring and represent $CH_2CH_2OCH_2CH_2$, $CH_2CH_2NR^5CH_2CH_2$ or $(CH_2)_{3-6}$.

**3.** Substituted 4-aminocyclohexanols according to claim 1, wherein $R^1$ and $R^2$ are selected independently of one another from H; $C_{1-8}$ alkyl or $C_{3-8}$ cycloalkyl, respectively saturated or unsaturated, branched or linear, singly or multiply substituted or unsubstituted; aryl or heteroaryl, respectively singly or multiply substituted or unsubstituted; or $C_1$-$C_3$-alkylene-bound aryl, $C_{3-8}$ cycloalkyl or heteroaryl, respectively singly or multiply substituted or unsubstituted; wherein $R^1$ and $R^2$ may not both be H.

4. Substituted 4-aminocyclohexanols according to claim 1, wherein $R^3$ is selected from heteroaryl, respectively unsubstituted or singly or multiply substituted.

5. Substituted 4-aminocyclohexanols according to claim 1, wherein $R^3$ is selected from aryl, unsubstituted or singly or multiply substituted.

6. Substituted 4-aminocyclohexanols according to claim 1, wherein the radicals $R^1$ and $R^2$ together form a ring and represent $CH_2CH_2OCH_2CH_2$, $CH_2CH_2NR^5CH_2CH_2$ or $(CH_2)_{3-6}$ and $R^3$ is selected from aryl, respectively unsubstituted or singly or multiply substituted.

7. Substituted 4-aminocyclohexanols according to claim 1, wherein $R^1$ and $R^2$ are selected independently of one another from H; $C_{1-8}$ alkyl or $C_{3-8}$ cycloalkyl, respectively saturated or unsaturated, branched or linear, singly or multiply substituted or unsubstituted; aryl or heteroaryl, respectively singly or multiply substituted or unsubstituted; or $C_1$-$C_3$-alkylene-bound aryl, $C_{3-8}$ cycloalkyl or heteroaryl, respectively singly or multiply substituted or unsubstituted; wherein $R^1$ and $R^2$ may not both be H and $R^3$ is selected from heteroaryl, respectively unsubstituted or singly or multiply substituted.

8. Substituted 4-aminocyclohexanols according to claim 1, wherein $R^7$ is selected from heteroaryl, respectively unsubstituted or singly or multiply substituted.

9. Substituted 4-aminocyclohexanols according to claim 1, wherein $R^4$ is selected from $-CHR^6R^7$, $-CHR^6-CH_2R^7$, $-CHR^6-CH_2--CH_2R^7$ or $-CHR^6-CH_2-CH_2-CH_2R^7$, where $R^6$ is selected from C(O)O-$C_{1-6}$ alkyl, saturated or unsaturated, branched or linear, singly or multiply substituted or unsubstituted.

10. Substituted 4-aminocyclohexanols according to claim 1, wherein $R^4$ is selected from $-C(Y)R^7$, $-C(Y)-CH_2R^7$, $-C(Y)-CH_2-CH_2R^7$ or $-C(Y)-CH_2-CH_2-CH_2R^7$, where Y = O or S.

11. Substituted 4-aminocyclohexanols according to any of claims 1, 4, 5, 8, 9 or 10, **characterised in that**
$R^1$ and $R^2$ independently of one another are selected from H; saturated or unsaturated, branched or unbranched, singly or multiply substituted or unsubstituted $C_{1-8}$-alkyl; wherein $R^1$ and $R^2$ may not both be H,
or the radicals $R^1$ and $R^2$ together form a ring and represent $CH_2CH_2OCH_2CH_2$, $CH_2CH_2NR^5CH_2CH_2$ or $(CH_2)_{3-6}$, where $R^5$ is selected from H; saturated or unsaturated, branched or unbranched, singly or multiply substituted or unsubstituted $C_{1-8}$-alkyl,
preferably
$R^1$ and $R^2$ independently of one another are selected from H; saturated or unsaturated, branched or unbranched, singly or multiply substituted or unsubstituted $C_{1-4}$-alkyl; wherein $R^1$ and $R^2$ may not both be H,
or the radicals $R^1$ and $R^2$ together form a ring and represent $(CH_2)_{4-5}$,
in particular
$R^1$ and $R^2$ independently of one another are selected from methyl or ethyl or the radicals $R^1$ and $R^2$ together form a ring and represent $(CH_2)_5$.

12. Substituted 4-aminocyclohexanols according to any of claims 3 or 7,
**characterised in that**
$R^1$ and $R^2$ independently of one another are selected from H; saturated or unsaturated, branched or unbranched, singly or multiply substituted or unsubstituted $C_{1-8}$-alkyl; wherein $R^1$ and $R^2$ may not both be H,
preferably
$R^1$ and $R^2$ independently of one another are selected from H; saturated or unsaturated, branched or unbranched, singly or multiply substituted or unsubstituted $C_{1-4}$-alkyl; wherein $R^1$ and $R^2$ may not both be H,
in particular
$R^1$ and $R^2$ independently of one another are selected from methyl or ethyl.

13. Substituted 4-aminocyclohexanols according to any of claims 2 or 6,
**characterised in that**
$R^1$ and $R^2$ together form a ring and represent $CH_2CH_2OCH_2CH_2$, $CH_2CH_2NR^5CH_2CH_2$ or $(CH_2)_{3-6}$,
where $R^5$ is selected from H; saturated or unsaturated, branched or unbranched, singly or multiply substituted or unsubstituted $C_{1-8}$-alkyl,
preferably
$R^1$ and $R^2$ together form a ring and represent $(CH_2)_{4-5}$,

in particular
$R^1$ and $R^2$ together form a ring and represent $(CH_2)_5$.

**14.** Substituted 4-aminocyclohexanols according to any of claims 1, 2, 3, 8, 9 or 10, **characterised in that**
$R^3$ is selected from respectively unsubstituted or singly or multiply substituted phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl;
preferably
$R^3$ is selected from respectively unsubstituted or singly or multiply substituted phenyl, thiophenyl, pyridyl, furyl, benzofuranyl, benzodioxolanyl, indolyl, indanyl, benzodioxanyl, pyrrolyl, pyrimidyl or pyrazinyl;
in particular
$R^3$ is selected from respectively unsubstituted or singly or multiply substituted phenyl, pyridyl, furyl or thiophenyl.

**15.** Substituted 4-aminocyclohexanols according to any of claims 4 or 7,
**characterised in that**
$R^3$ is selected from respectively unsubstituted or singly or multiply substituted thiophenyl, benzothiophenyl, pyridyl, furyl, benzofuranyl, benzodioxolanyl, indolyl, indanyl, benzodioxanyl, pyrrolyl, pyrimidyl or pyrazinyl;
preferably
$R^3$ is selected from respectively unsubstituted or singly or multiply substituted thiophenyl, pyridyl, furyl, benzofuranyl, benzodioxolanyl, indolyl, indanyl, benzodioxanyl, pyrrolyl, pyrimidyl or pyrazinyl;
in particular
$R^3$ is selected from respectively unsubstituted or singly or multiply substituted pyridyl, furyl or thiophenyl.

**16.** Substituted 4-aminocyclohexanols according to any of claims 5 or 6,
**characterised in that**
$R^3$ is selected from phenyl, naphthyl, anthracenyl;
preferably
$R^3$ is selected from respectively unsubstituted or singly or multiply substituted phenyl or naphthyl;
in particular
$R^3$ is selected from unsubstituted or singly or multiply substituted phenyl.

**17.** Substituted 4-aminocyclohexanols according to any of claims 1 to 7,
**characterised in that**
$R^4$ is selected from respectively unsubstituted or singly or multiply substituted $C_{3-8}$-cycloalkyl, aryl or heteroaryl; or $-R^8-L-R^9$
preferably
$R^4$ is selected from respectively unsubstituted or singly or multiply substituted cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl; or $-R^8-L-R^9$
in particular
$R_4$ is selected from respectively unsubstituted or singly or multiply substituted cyclopentyl, cyclohexyl, cycloheptyl, cyclooctyl, anthracenyl, indolyl, naphthyl, benzofuranyl, benzothiazolyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl; or $-R^8-L-R^9$.

**18.** Substituted 4-aminocyclohexanols according to claim 17, **characterised in that**
$R^8$ is selected from
respectively unsubstituted or singly or multiply substituted indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl,
L is selected from
-C(O)-NH-, -NH-C(O)-, -C(O)-O-, -O-C(O)-, -O-, -S-or $-S(O)_2-$,
and/or $R^9$ is selected from

respectively unsubstituted or singly or multiply substituted indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl,
preferably
$R^8$ is selected from
respectively unsubstituted or singly or multiply substituted indolyl, benzothiophenyl, phenyl, thiophenyl, furyl, pyridyl, pyrrolyl, pyrazinyl or pyrimidyl,
L is selected from
-C(O)-NH-, -NH-C(O)-, -C(O)-O-, -O-C(O)-or $-S(O)_2-$,
and/or $R^9$ is selected from
respectively unsubstituted or singly or multiply substituted indolyl, benzothiophenyl, phenyl, thiophenyl, furyl, pyridyl, pyrrolyl, pyrazinyl or pyrimidyl,
in particular
$R^8$ is selected from
unsubstituted indolyl,
L is selected from

$-S(O)_2$

and $R^9$ is selected from
unsubstituted phenyl.

**19.** Substituted 4-aminocyclohexanols according to any of claims 1 to 7,
**characterised in that**
$R^4$ is selected from $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$
where Y = O, S or $H_2$,
preferably
$R^4$ is selected from $-CHR^6R^7$, $-CHR^6-CH_2R^7$, $-CHR^6-CH_2-CH_2R^7$, $-C(Y)R^7$, $-C(Y)-CH_2R^7$ or $-C(Y)-CH_2-CH_2R^7$
where Y = O or S,
in particular
$R^4$ is selected from $-CHR^6R^7$, $-CHR^6-CH_2R^7$, $-C(Y)R^7$ or $-C(Y)-CH_2R^7$
where Y = O.

**20.** Substituted 4-aminocyclohexanols according to claim 19, **characterised in that**
$R^6$ is selected from
H, saturated or unsaturated, branched or unbranched, singly or multiply substituted or unsubstituted $C_{1-4}$-alkyl; or saturated or unsaturated, branched or unbranched, singly or multiply substituted or unsubstituted $C(O)O-C_{1-4}$-alkyl;
preferably
H, saturated or unsaturated, branched or unbranched, singly or multiply substituted or unsubstituted $C_{1-4}$-alkyl;
in particular
H, $CH_3$ and $C_2H_5$.

**21.** Substituted 4-aminocyclohexanols according to claim 19, **characterised in that**
$R^7$ is selected from respectively unsubstituted or singly or multiply substituted $C_{3-8}$-cycloalkyl, aryl or heteroaryl;
preferably
$R^7$ is selected from respectively unsubstituted or singly or multiply substituted cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl;
in particular
$R^7$ is selected from respectively unsubstituted or singly or multiply substituted cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl.

**22.** Substituted 4-aminocyclohexanols according to claim 8, **characterised in that** $R^4$ is selected from respectively unsubstituted or singly or multiply substituted heteroaryl; or $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$; or - $R^8$-L-$R^9$
where Y = $H_2$,
preferably
$R^4$ is selected from respectively unsubstituted or singly or multiply substituted indolyl, benzofuranyl, benzothiophenyl, indanyl, benzodioxanyl, benzodioxolanyl, acenaphthyl, carbazolyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl; or $-CHR^6R^7$, $-CHR^6-CH_2R^7$, $-CHR^6-CH_2-CH_2R^7$, $-C(Y)R^7$, $-C(Y)-CH_2R^7$ or $-C(Y)-CH_2-CH_2R^7$
where Y = $H_2$,
in particular
$R^4$ is selected from respectively unsubstituted or singly or multiply substituted indolyl, benzofuranyl, benzothiophenyl, benzothiazolyl, indanyl, benzodioxanyl, benzodioxolanyl, acenaphthyl, carbazolyl, thiophenyl, furyl, pyridyl, pyrrolyl, pyrazinyl or pyrimidyl; or $-CHR^6R^7$, $-CHR^6-CH_2R^7$, $-C(Y)R^7$ or $-C(Y)-CH_2R^7$,
where Y = $H_2$.

**23.** Substituted 4-aminocyclohexanols according to claim 22, **characterised in that**
$R^6$ is selected from
H, saturated or unsaturated, branched or unbranched, singly or multiply substituted or unsubstituted $C_{1-4}$-alkyl;
preferably
H, saturated or unsaturated, branched or unbranched, singly or multiply substituted or unsubstituted $C_{1-4}$-alkyl;
in particular
H, $CH_3$ and $C_2H_5$
and/or
$R^7$ is selected from respectively unsubstituted or singly or multiply substituted indolyl, benzofuranyl, benzothiophenyl, indanyl, benzodioxanyl, benzodioxolanyl, acenaphthyl, carbazolyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl;
preferably
$R^7$ is selected from respectively unsubstituted or singly or multiply substituted indolyl, benzofuranyl, benzothiophenyl, indanyl, benzodioxanyl, benzodioxolanyl, acenaphthyl, carbazolyl, thiophenyl, furyl, pyridyl, pyrrolyl, pyrazinyl or pyrimidyl;
in particular
$R^7$ is selected from respectively unsubstituted or singly or multiply substituted thiophenyl, furyl, pyridyl, pyrrolyl, pyrazinyl or pyrimidyl.

**24.** Substituted 4-aminocyclohexanols according to claim 22, **characterised in that** $R^8$ is selected from respectively unsubstituted or singly or multiply substituted indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl,
L is selected from
-C(O)-NH-, -NH-C(O)-, -C(O)-O-, -O-C(O)-, -O-, -S-or $-S(O)_2-$,
and/or $R^9$ is selected from
respectively unsubstituted or singly or multiply substituted indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl,
preferably
$R^8$ is selected from
respectively unsubstituted or singly or multiply substituted indolyl, benzothiophenyl, phenyl, thiophenyl, furyl, pyridyl, pyrrolyl, pyrazinyl or pyrimidyl,
L is selected from

-C(O)-NH-, -NH-C(O)-, -C(O)-O-, -O-C(O)-or -S(O)$_2$-,
and/or $R^9$ is selected from
respectively unsubstituted or singly or multiply substituted indolyl, benzothiophenyl, phenyl, thiophenyl, furyl, pyridyl, pyrrolyl, pyrazinyl or pyrimidyl,
in particular
$R^8$ is selected from
unsubstituted indolyl,
L is selected from

-S(O)$_2$-

and $R^9$ is selected from
unsubstituted phenyl.

**25.** Substituted 4-aminocyclohexanols according to claim 9, **characterised in that**
$R^4$ is selected from -CHR$^6$R$^7$, -CHR$^6$-CH$_2$R$^7$ or -CHR$^6$-CH$_2$-CH$_2$R$^7$
preferably
$R^4$ is selected from -CHR$^6$R$^7$ or -CHR$^6$-CH$_2$R$^7$,
in particular
$R^4$ is selected from -CHR$^6$R$^7$.

**26.** Substituted 4-aminocyclohexanols according to claim 25, **characterised in that**
$R^6$ is selected from
saturated or unsaturated, branched or unbranched, singly or multiply substituted or unsubstituted C(O)O-C$_{1-4}$-alkyl;
preferably
saturated or unsaturated, branched or unbranched, singly or multiply substituted or unsubstituted C(O)O-C$_{1-3}$-alkyl;
in particular
C(O)O-CH$_3$ and C(O)O-C$_2$H$_5$
and/or
$R^7$ is selected from respectively unsubstituted or singly or multiply substituted C$_{3-8}$-cycloalkyl, aryl or heteroaryl;
preferably
$R^7$ is selected from respectively unsubstituted or singly or multiply substituted cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl;
in particular
$R^7$ is selected from respectively unsubstituted or singly or multiply substituted cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl.

**27.** Substituted 4-aminocyclohexanols according to claim 10, **characterised in that**
$R^4$ is selected from -C(Y)R$^7$, -C(Y)-CH$_2$R$^7$, -C(Y)CH$_2$-CH$_2$R$^7$ or -C(Y)-CH$_2$-CH$_2$-CH$_2$R$^7$
where Y = O
preferably
$R^4$ is selected from -C(Y)R$^7$, -C(Y)-CH$_2$R$^7$ or -C(Y)-CH$_2$-CH$_2$R$^7$,
where Y = O
in particular
$R^4$ is selected from -C(Y)R$^7$ or -C(Y)-CH$_2$R$^7$,
where Y = O.

**28.** Substituted 4-aminocyclohexanols according to claim 27, **characterised in that**
$R^7$ is selected from respectively unsubstituted or singly or multiply substituted C$_{3-8}$-cycloalkyl, aryl or heteroaryl;
preferably
$R^7$ is selected from respectively unsubstituted or singly or multiply substituted cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl;
in particular
$R^7$ is selected from respectively unsubstituted or singly or multiply substituted cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl.

**29.** Substituted 4-aminocyclohexanols according to any of claims 1 to 28, **characterised in that** they are selected from the following group:

- 4-dimethylamino-1-(1-methyl-1H-indol-2-yl)-4-phenylcyclohexanol
- 1-benzo[b]thiophen-2-yl-4-dimethylamino-4-phenylcyclohexanol
- 1-benzo[b]thiophen-3-yl-4-dimethylamino-4-phenylcyclohexanol
- 1-(1-benzenesulphonyl-1H-indol-2-yl)-4-dimethylamino-4-phenylcyclohexanol
- 1-benzofuran-2-yl-4-dimethylamino-4-phenylcyclohexanol
- 1-benzothiazol-2-yl-4-dimethylamino-4-phenylcyclohexanol

optionally in the form of their racemates, their pure stereoisomers, in particular enantiomers or diastereomers, or in the form of mixtures of stereoisomers, in particular the enantiomers or diastereomers, in any mixing ratio; in the prepared form or in the form of their acids or bases or in the form of their salts, in particular the physiologically acceptable salts or in the form of their solvates, in particular the hydrates.

**30.** Pharmaceutical composition containing at least one substituted 4-aminocyclohexanol according to any of claims 1 to 29, optionally in the form of its racemate, the pure stereoisomers, in particular enantiomers or diastereomers, or in the form of mixtures of stereoisomers, in particular enantiomers or diastereomers, in any mixing ratio; in the prepared form or in the form of the acids or bases or in the form of the salts, in particular the physiologically acceptable salts or in the form of their solvates, in particular the hydrates; as well as optionally suitable additives and/or auxiliaries and/or optionally further active ingredients.

**31.** Pharmaceutical composition according to claim 30, **characterised in that**, in addition to at least one substituted 4-aminocyclohexanol, the pharmaceutical composition also contains an opioid, preferably a strong opioid, in particular morphine, or an anaesthetic, preferably hexobarbital or halothane.

**32.** Use of a substituted 4-aminocyclohexanol according to any of claims 1 to 29 optionally in the form of its racemate, the pure stereoisomers, in particular enantiomers or diastereomers, or in the form of mixtures of stereoisomers, in particular enantiomers or diastereomers, in any mixing ratio; in the prepared form or in the form of the acids or bases or in the form of the salts, in particular the physiologically acceptable salts or in the form of the solvates, in particular the hydrates; for producing a pharmaceutical composition for the treatment of pain, in particular of acute, visceral, neuropathic or chronic pain; of migraines or locomotive disorders; as an anticonvulsant or muscle relaxant.

**33.** Use of a substituted 4-aminocyclohexanol of general formula I,

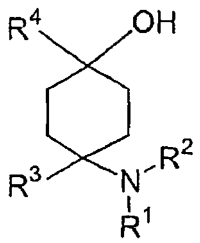

I

wherein
$R^1$ and $R^2$ independently of one another are selected from H; respectively saturated or unsaturated, branched or

unbranched, singly or multiply substituted or unsubstituted $C_{1-8}$-alkyl or $C_{3-8}$-cycloalkyl; respectively singly or multiply substituted or unsubstituted aryl or heteroaryl; or respectively singly or multiply substituted or unsubstituted $C_{1-3}$-alkylene-bound aryl, $C_{3-8}$-cycloalkyl or heteroaryl;
wherein $R^1$ and $R^2$ may not both be H,
or the radicals $R^1$ and $R^2$ together form a ring and represent $CH_2CH_2OCH_2CH_2$, $CH_2CH_2NR^5CH_2CH_2$ or $(CH_2)_{3-6}$, where $R^5$ is selected from H; respectively saturated or unsaturated, branched or unbranched, singly or multiply substituted or unsubstituted $C_{1-8}$-alkyl or $C_{3-8}$-cycloalkyl; respectively singly or multiply substituted or unsubstituted aryl or heteroaryl; or respectively singly or multiply substituted or unsubstituted $C_{1-3}$-alkylene-bound aryl, $C_{3-8}$-cycloalkyl or heteroaryl;
$R^3$ is selected from respectively unsubstituted or singly or multiply substituted aryl or heteroaryl;
$R^4$ is selected from respectively unsubstituted or singly or multiply substituted $C_{3-8}$-cycloalkyl, aryl or heteroaryl; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$; or $-R^8-L-R^9$
where Y = O, S or $H_2$,
where $R^6$ is selected from
H, saturated or unsaturated, branched or unbranched, singly or multiply substituted or unsubstituted $C_{1-7}$-alkyl; or saturated or unsaturated, branched or unbranched, singly or multiply substituted or unsubstituted $C(O)O-C_{1-6}$-alkyl;
where $R^7$ is selected from
H; respectively unsubstituted or singly or multiply substituted $C_{3-8}$-cycloalkyl, aryl or heteroaryl,
where $R^8$ is selected from
respectively unsubstituted or singly or multiply substituted aryl or heteroaryl,
where L is selected from
-C(O)-NH-, -NH-C(O)-, -C(O)-O-, -O-C(O)-, -O-, -S- or $-S(O)_2-$
where $R^9$ is selected from
respectively unsubstituted or singly or multiply substituted aryl or heteroaryl,
wherein "singly or multiply substituted aryl or heteroaryl" means "aryl or heteroaryl singly or multiply substituted with $R^{82}$, $OR^{82}$, halogen, $CF_3$, CN, $NO_2$, $NR^{83}R^{84}$, $C_{1-6}$ alkyl, $C_{1-6}$ alkoxy, $C_{3-8}$ cycloalkoxy, $C_{3-8}$ cycloalkyl or $C_{2-6}$ alkylene",
wherein
$R^{82}$ represents H, $C_{1-10}$ alkyl, aryl, heteroaryl or an aryl or heteroaryl radical which is bound via $C_{1-3}$ alkyl, saturated or unsaturated, or a $C_{1-3}$ alkylene group, wherein these aryl and heteroaryl radicals may not themselves be substituted with aryl or heteroaryl radicals;
$R^{83}$ and $R^{84}$ are the same or different and represent H, $C_{1-10}$ alkyl, aryl, heteroaryl or an aryl or heteroaryl radical which is bound via $C_{1-3}$ alkyl, saturated or unsaturated, or a $C_{1-3}$ alkylene group, wherein these aryl and heteroaryl radicals may not themselves be substituted with aryl or heteroaryl radicals,
or the radicals $R^{83}$ and $R^{84}$ together represent $CH_2CH_2OCH_2CH_2$, $CH_2CH_2NR^{85}CH_2CH_2$ or $(CH_2)_{3-6}$, and the radical $R^{85}$ represents H, $C_{1-10}$ alkyl, aryl, heteroaryl or an aryl or heteroaryl radical which is bound via $C_{1-3}$ alkyl, saturated or unsaturated, or a $C_{1-3}$ alkylene group, wherein these aryl and heteroaryl radicals may not themselves be substituted with aryl or heteroaryl radicals;
"singly or multiply substituted cycloalkyl" means "cycloalkyl singly or multiply substituted with F, Cl, Br, I, $NH_2$, SH, OH, $OC_{1-3}$ alkyl or $C_{1-3}$ alkyl",
and
"singly or multiply substituted alkyl" means "alkyl singly or multiply substituted with F, Cl, Br, I, $NH_2$, SH or OH", optionally in the form of its racemate, the pure stereoisomers, in particular enantiomers or diastereomers, or in the form of mixtures of stereoisomers, in particular enantiomers or diastereomers, in any mixing ratio; in the prepared form or in the form of the acids or bases or in the form of the salts, in particular the physiologically acceptable salts or in the form of the solvates, in particular the hydrates; for producing a pharmaceutical composition for the treatment of phobias, stress and syndromes associated with stress, depression, epilepsy, Alzheimer's disease, senile dementia, general cognitive dysfunction, learning difficulties and memory loss (as a nootropic), withdrawal symptoms, alcohol and/or drug and/or medicine abuse and/or dependency, sexual dysfunction, cardiovascular diseases, hypotension, hypertension, tinnitus, pruritus, migraine, defective hearing, defective bowel motility, impaired assimilation of food, anorexia, obesity, locomotive disorders, diarrhoea, cachexia, urinary incontinence or as a muscle relaxant, anticonvulsant, antitussive or anaesthetic or for co-administration during treatment with an opioid analgaesic or with an anaesthetic, for diuresis or antinatriuresis and/or anxiolysis.

**34.** Method of producing a substituted 4-aminocyclohexanol according to any of claims 1 to 29 comprising the following steps:

a. a cyclohexane-1,4-dione protected by the groups $S^1$ and $S^2$ according to formula II is reacted in the presence of a compound of formula $HNR^{01}R^{02}$ with a cyanide, preferably potassium cyanide, to form a protected N-substituted 1-amino-4-oxo-cyclohexanecarbonitrile derivative according to formula III;

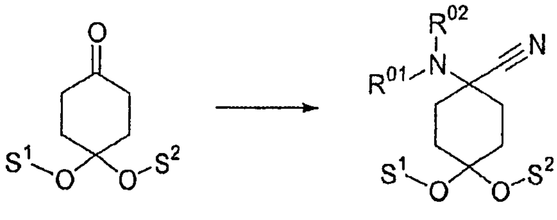

II III

optionally, the compound is then acylated, alkylated or sulphonated in any sequence and optionally repeatedly and/or, in the case of compounds where $R^{01}$ and/or $R^{02}$ and/or $R^{06}$ = H protected by a protective group, a protective group is cleaved off at least once and optionally acylated, alkylated or sulphonated and/or, in the case of a compound where $R^{01}$ and/or $R^{02}$ and/or $R^{06}$ = H, a protective group is introduced at least once and optionally acylated, alkylated or sulphonated,

b. the aminonitrile according to formula III is reacted with organometallic reagents, preferably Grignard or organolithium reagents, having the formula metal-$R^3$ to form a compound according to formula IVa;

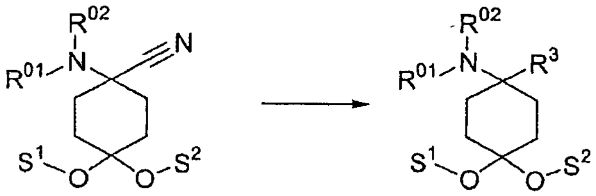

III IVa

optionally, the compound is then acylated, alkylated or sulphonated in any sequence and optionally repeatedly and/or, in the case of compounds where $R^{01}$ and/or $R^{02}$ and/or $R^{06}$ = H protected by a protective group, a protective group is cleaved off at least once and optionally acylated, alkylated or sulphonated and/or, in the case of a compound where $R^{01}$ and/or $R^{02}$ and/or $R^{06}$ = H, a protective group is introduced at least once and optionally acylated, alkylated or sulphonated,

c. the protective groups $S^1$ and $S^2$ are cleaved off according to formula III on the compound according to formula IVa to form a 4-substituted 4-aminocyclohexanone derivative according to formula IV;

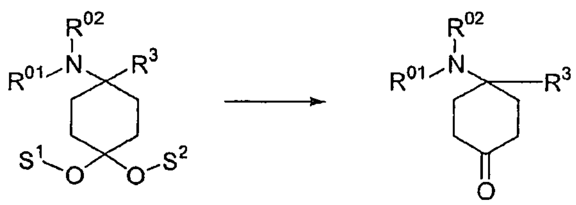

IVa IV

optionally, the compound is then acylated, alkylated or sulphonated in any sequence and optionally repeatedly

and/or, in the case of compounds where $R^{01}$ and/or $R^{02}$ and/or $R^{06}$ = H protected by a protective group, a protective group is cleaved off at least once and optionally acylated, alkylated or sulphonated and/or, in the case of a compound where $R^{01}$ and/or $R^{02}$ and/or $R^{06}$ = H, a protective group is introduced at least once and optionally acylated, alkylated or sulphonated until a compound according to formula I is obtained,

d. the 4-substituted 4-aminocyclohexanone derivative according to formula IV is reacted with organometallic reagents, preferably Grignard or organolithium reagents, having the formula metal-$R^{04}$ to form a compound according to formula V;

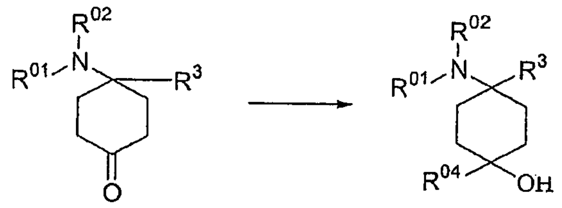

IV V

optionally, the compound is then acylated, alkylated or sulphonated in any sequence and optionally repeatedly and/or, in the case of compounds where $R^{01}$ and/or $R^{02}$ and/or $R^{04}$ and/or $R^{05}$ and/or $R^{06}$ = H protected by a protective group, a protective group is cleaved off at least once and optionally acylated, alkylated or sulphonated and/or, in the case of a compound where $R^{01}$ and/or $R^{02}$ and/or $R^{04}$ and/or $R^{05}$ and/or $R^{06}$ = H, a protective group is introduced at least once and optionally acylated, alkylated or sulphonated,

wherein $R^1$, $R^2$, $R^3$ and $R^4$ have the meaning given in claim 1
and
$R^{01}$ and $R^{02}$ independently of one another are selected from H; H provided with a protective group; respectively saturated or unsaturated, branched or unbranched, singly or multiply substituted or unsubstituted $C_{1-8}$-alkyl or $C_{3-8}$-cycloalkyl; respectively singly or multiply substituted or unsubstituted aryl or heteroaryl; or respectively singly or multiply substituted or unsubstituted $C_{1-3}$-alkylene-bound aryl, $C_{3-8}$-cycloalkyl or heteroaryl;
or the radicals $R^{01}$ and $R^{02}$ together form a ring and represent $CH_2CH_2OCH_2CH_2$, $CH_2CH_2NR^{05}CH_2CH_2$ or $(CH_2)_{3-6}$,
where $R^{05}$ is selected from H; H provided with a protective group; respectively saturated or unsaturated, branched or unbranched, singly or multiply substituted or unsubstituted $C_{1-8}$-alkyl or $C_{3-8}$-cycloalkyl; respectively singly or multiply substituted or unsubstituted aryl or heteroaryl; or respectively singly or multiply substituted or unsubstituted $C_{1-3}$-alkylene-bound aryl, $C_{3-8}$-cycloalkyl or heteroaryl;
$R^{04}$ is selected from H, H provided with a protective group; respectively unsubstituted or singly or multiply substituted $C_{3-8}$-cycloalkyl, aryl or heteroaryl; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$; or $-R^8-L-R^9$
where Y = O, S or $H_2$,
where $R^6$ is selected from
H, saturated or unsaturated, branched or unbranched, singly or multiply substituted or unsubstituted $C_{1-7}$-alkyl;
and where $R^7$ is selected from
H; respectively unsubstituted or singly or multiply substituted $C_{3-8}$-cycloalkyl, aryl or heteroaryl,
where $R^8$ is selected from
respectively unsubstituted or singly or multiply substituted aryl or heteroaryl,
where L is selected from
-C(O)-NH-, -NH-C(O)-, -C(O)-O-, -O-C(O)-, -O-, -S-or $-S(O)_2-$
where $R^9$ is selected from
respectively unsubstituted or singly or multiply substituted aryl or heteroaryl, and $S^1$ and $S^2$ independently of one another are selected from protective groups or together represent a protective group, preferably monoacetal.

**35.** Method of producing a substituted 4-aminocyclohexanol according to claim 34, **characterised in that** the protective groups on the H when $R^{01}$, $R^{02}$, $R^{04}$ and/or $R^{05}$ are selected from alkyl, benzyl or carbamates, for example FMOC, Z or Boc.

## Revendications

**1.** 4-aminocyclohexanols substitués de formule générale I,

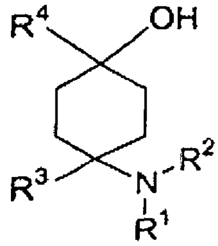

I

dans laquelle
$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ; $R^1$ et $R^2$ ne pouvant pas représenter tous deux H,
ou bien les restes $R^1$ et $R^2$ forment ensemble un noyau et représentent $CH_2CH_2OCH_2CH_2$, $CH_2CH_2NR^5CH_2CH_2$ ou $(CH_2)_{3-6}$,
$R^5$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ;
$R^3$ est choisi entre un reste aryle ou un reste hétéroaryle, chacun non substitué ou substitué une ou plusieurs fois ;
$R^4$ est choisi entre un reste cycloalkyle en $C_3$ à $C_8$, aryle ou hétéroaryle, chacun non substitué ou substitué une ou plusieurs fois ; un groupe $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ ou $-C(Y)-CH_2-CH_2-CH_2R^7$; ou $-R^8-L-R^{9'}$ Y représentant O, S ou $H_2$,
$R^6$ étant choisi entre H, un reste alkyle en $C_1$ à $C_7$, saturé ou non saturé, ramifié ou non ramifié, substitué une ou plusieurs fois ou non substitué ; ou un groupe C(O)O-(alkyle en $C_1$ à $C_6$), saturé ou non saturé, ramifié ou non ramifié, substitué une ou plusieurs fois ou non substitué ;
$R^7$ étant choisi entre H ; un reste cycloalkyle en $C_3$ à $C_8$, aryle ou hétéroaryle, chacun non substitué ou substitué une ou plusieurs fois,
$R^8$ étant choisi entre un reste aryle ou hétéroaryle, chacun non substitué ou substitué une ou plusieurs fois,
L étant choisi entre -C(O)-NH-, -NH-C(O)-, -C(O)-O-, -O-C(O)-, -O-, -S- ou $-S(O)_2-$
$R^9$ étant choisi entre un reste aryle ou hétéroaryle, chacun non substitué ou substitué une ou plusieurs fois,
sous réserve que lorsque $R^3$ représente un reste phényle, substitué ou non substitué et $R^4$ représente un reste phényle ou un groupe $-CHR^6R^7$, $-CHR^6-CH_2R^7$, $-CHR^5-CH_2-CH_2R^7$, $-CHR^5-CH_2-CH_2-CH_2R^7$, $C(Y)R^7$, $-C(Y)-CH_2R^7$, $-C(Y)-CH_2-CH_2R^7$ ou $-C(Y)-CH_2-CH_2-CH_2R^7$ avec Y = $H_2$,
$R^6$ = H, un reste alkyle en $C_1$ à $C_5$, saturé ou non saturé, ramifié ou non ramifié, substitué une ou plusieurs fois ou non substitué,
et/ou
$R^7$ = H, un reste cycloalkyle en $C_3$ à $C_8$ ou phényle, chacun substitué ou non substitué,
$R^1$ et $R^2$ ne représentent pas tous deux indépendamment l'un de l'autre un reste alkyle en $C_1$ à $C_5$,
lorsque $R^3$ est un reste thiophényle, substitué ou non substitué, et $R^4$ est un reste $-CH_2-CH_2$-phényle, les restes $R^1$ et $R^2$ ne forment pas ensemble un noyau et ne représentent pas ensemble $(CH_2)_5$,
l'expression "aryle ou hétéroaryle substitué une ou plusieurs fois" désigne "un reste aryle ou hétéroaryle substitué une ou plusieurs fois avec $R^{82}$, $OR^{82}$, un halogène, un radical $CF_3$, CN, $NO_2$, $NR^{83}R^{84}$, alkyle en $C_1$ à $C_6$, alkoxy en $C_1$ à $C_6$, cycloalkoxy en $C_3$ à $C_8$, cycloalkyle en $C_3$ à $C_8$ ou alkylène en $C_2$ à $C_6$",
$R^{82}$ représentant H, un reste alkyle en $C_1$ à $C_{10}$, aryle, hétéroaryle ou un reste aryle ou hétéroaryle lié par l'intermédiaire d'un groupe alkyle en $C_1$ à $C_3$, saturé ou non saturé, ou par l'intermédiaire d'un groupe alkylène en $C_1$ à $C_3$, ces restes aryle et hétéroaryle ne pouvant pas eux-mêmes être substitués avec des restes aryle ou hétéroaryle ;
$R^{83}$ et $R^{84}$, identiques ou différents, représentant H, un reste alkyle en $C_1$ à $C_{10}$, aryle, hétéroaryle ou un reste aryle ou hétéroaryle lié par l'intermédiaire d'un groupe alkyle en $C_1$ à $C_3$, saturé ou non saturé, ou d'un groupe alkylène

en $C_1$ à $C_3$, ces restes aryle et hétéroaryle ne pouvant pas eux-mêmes être substitués avec des restes aryle ou hétéroaryle,
ou bien les restes $R^{83}$ et $R^{84}$ représentent ensemble un groupe $CH_2CH_2OCH_2CH_2$, $CH_2CH_2NR^{85}CH_2CH_2$ ou $(CH_2)_{3-6}$, et le reste $R^{85}$ représentant H, un reste alkyle en $C_1$ à $C_{10}$, aryle, hétéroaryle ou un reste aryle ou hétéroaryle lié par l'intermédiaire d'un groupe alkyle en $C_1$ à $C_3$, saturé ou non saturé, ou d'un groupe alkylène en $C_1$ à $C_3$, ces restes aryle et hétéroaryle ne pouvant pas être substitués eux-mêmes avec des restes aryle ou hétéroaryle ; "cycloalkyle substitué une ou plusieurs fois" désigne "un reste cycloalkyle substitué une ou plusieurs fois avec F, Cl, Br, I, $NH_2$, SH, OH, O(alkyle en $C_1$ à $C_3$) ou alkyle en $C_1$ à $C_3$" ;
et
"alkyle substitué une ou plusieurs fois" désigne "un groupe alkyle substitué une ou plusieurs fois avec F, Cl, Br, I, $NH_2$, SH ou OH",
éventuellement sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates.

**2.** 4-aminocyclohexanols substitués de formule 1, dans laquelle les restes $R^1$ et $R^2$ forment ensemble un noyau et représentent $CH_2CH_2OCH_2CH_2$, $CH_2CH_2NR^5CH_2CH_2$ ou $(CH_2)_{3-6}$.

**3.** 4-aminocyclohexanols substitués suivant la revendication 1, dans lesquels $R^1$ et $R^2$ sont choisis indépendamment l'un de l'autre entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ; $R^1$ et $R^2$ ne pouvant pas représenter tous deux H.

**4.** 4-aminocyclohexanols substitués suivant la revendication 1, dans lesquels $R^3$ est choisi entre des restes hétéroaryle, chacun non substitué ou substitué une
ou plusieurs fois.

**5.** 4-aminocyclohexanols substitués suivant la revendication 1, dans lesquels $R^3$ est choisi entre des restes aryle non substitués ou substitués une ou plusieurs fois.

**6.** 4-aminocyclohexanols substitués suivant la revendication 1, dans lesquels les restes $R^1$ et $R^2$ forment ensemble un noyau et représentent $CH_2CH_2OCH_2CH_2$, $CH_2CH_2NR^5CH_2CH_2$ ou $(CH_2)_{3-6}$ et $R^3$ est choisi entre des restes aryle, chacun non substitué ou substitué une ou plusieurs fois.

**7.** 4-aminocyclohexanols substitués suivant la revendication 1, dans lesquels $R^1$ et $R^2$ sont choisis indépendamment l'un de l'autre entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ;
ou un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ; $R^1$ et $R^2$ ne pouvant pas représenter tous deux H et $R^3$ est choisi entre des restes hétéroaryle non substitués ou substitués une ou plusieurs fois.

**8.** 4-aminocyclohexanols substitués suivant la revendication 1, dans lesquels $R^7$ est choisi entre des restes hétéroaryle, chacun non substitué ou substitué une
ou plusieurs fois.

**9.** 4-aminocyclohexanols substitués suivant la revendication 1, dans lesquels $R^4$ est choisi entre un reste $-CHR^6R^7$, $-CHR^6-CH_2R^7$, $-CHR^6-CH_2-CH_2R^7$ ou $-CHR^6-CH_2-CH_2-CH_2R^7$,
$R^6$ étant choisi entre des restes C(O)O(alkyle en $C_1$ à $C_6$) saturés ou non saturés, ramifiés ou non ramifiés, substitués une ou plusieurs fois ou non substitués.

**10.** 4-aminocyclohexanols substitués suivant la revendication 1, dans lesquels $R^4$ est choisi entre $-C(Y)R^7$, $-C (Y) -CH_2R^7$, $-C (Y) -CH_2-CH_2R^7$ ou $-C (Y) -CH_2-CH_2-CH_2R^7$,
Y représentant O ou S.

**11.** 4-aminocyclohexanols substitués suivant l'une des revendications 1, 4, 5, 8, 9 ou 10, **caractérisés en ce que** $R^1$ et $R^2$ sont choisis, indépendamment l'un de l'autre, entre H ; un reste alkyle en $C_1$ à $C_8$ saturé ou non saturé, ramifié ou non ramifié, substitué une ou plusieurs fois ou non substitué ; $R^1$ et $R^2$ ne pouvant pas représenter tous deux H, ou bien les restes $R^1$ et $R^2$ forment ensemble un noyau et représentent $CH_2CH_2OCH_2CH_2$, $CH_2CH_2NR^5CH_2CH_2$ ou $(CH_2)_{3-6}$,
$R^5$ étant choisi entre H ; un reste alkyle en $C_1$ à $C_8$, saturé ou non saturé, ramifié ou non ramifié, substitué une ou plusieurs fois ou non substitué, avantageusement
$R^1$ et $R^2$ sont choisis, indépendamment l'un de l'autre, entre H ; un reste alkyle en $C_1$ à $C_4$, saturé ou non saturé, ramifié ou non ramifié, substitué une ou plusieurs fois ou non substitué ; $R^1$ et $R^2$ ne pouvant pas représenter tous deux H, ou bien les restes $R^1$ et $R^2$ forment ensemble un noyau et représentent $(CH_2)_{4-5}$, en particulier
$R^1$ et $R^2$ sont choisis indépendamment l'un de l'autre entre un reste méthyle ou éthyle ou bien les restes $R^1$ et $R^2$ forment ensemble un noyau et représentent $(CH_2)_5$.

**12.** 4-aminocyclohexanols substitués suivant l'une des revendications 3 ou 7, **caractérisés en ce que**
$R^1$ et $R^2$ sont choisis, indépendamment l'un de l'autre, entre H ; un reste alkyle en $C_1$ à $C_8$, saturé ou non saturé, ramifié ou non ramifié, substitué une ou plusieurs fois ou non substitué ; $R^1$ et $R^2$ ne pouvant pas représenter tous deux H,
avantageusement
$R^1$ et $R^2$ sont choisis indépendamment l'un de l'autre entre H ; un reste alkyle en $C_1$ à $C_4$, saturé ou non saturé, ramifié ou non ramifié, substitué une ou plusieurs fois
ou non substitué ;
$R^1$ et $R^2$ ne pouvant pas représenter tous deux H,
en particulier
$R^1$ et $R^2$ sont choisis indépendamment l'un de l'autre entre un reste méthyle ou éthyle.

**13.** 4-aminocyclohexanols substitués suivant l'une des revendications 2 ou 6, **caractérisés en ce que**
$R^1$ et $R^2$ forment ensemble un noyau et représentent $CH_2CH_2OCH_2CH_2$, $CH_2CH_2NR^5CH_2CH_2$ ou $(CH_2)_{3-6}$,
$R^5$ étant choisi entre H ; un reste alkyle en $C_1$ à $C_8$, saturé ou non saturé, ramifié ou non ramifié, substitué une ou plusieurs fois ou non substitué,
avantageusement
$R^1$ et $R^2$ forment ensemble un noyau et représentent un groupe $(CH_2)_{4-5}$,
en particulier
$R^1$ et $R^2$ forment ensemble un noyau et représentent un groupe $(CH_2)_5$.

**14.** 4-aminocyclohexanols substitués suivant l'une des revendications 1, 2, 3, 8, 9 ou 10, **caractérisés en ce que**
$R^3$ est choisi entre un reste phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; avantageusement
$R^3$ est choisi entre un reste phényle, thiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ;
en particulier
$R^3$ est choisi entre un reste phényle, pyridyle, furyle ou thiophényle, chacun non substitué ou substitué une ou plusieurs fois.

**15.** 4-aminocyclohexanols substitués suivant l'une des revendications 4 ou 7, **caractérisés en ce que**
$R^3$ est choisi entre un reste thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ;
avantageusement
$R^3$ est choisi entre un reste thiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ;
en particulier
$R^3$ est choisi entre un reste pyridyle, furyle ou thiophényle, chacun non substitué ou substitué une ou plusieurs fois.

**16.** 4-aminocyclohexanols substitués suivant l'une des revendications 5 ou 6, **caractérisés en ce que**
$R^3$ est choisi entre un reste phényle, naphtyle, anthracényle :
avantageusement

$R^3$ est choisi entre un reste phényle ou naphtyle, chacun non substitué ou substitué une ou plusieurs fois ;
en particulier
$R^3$ est choisi entre un reste phényle non substitué ou un reste phényle substitué une ou plusieurs fois.

**17.** 4-aminocyclohexanols substitués suivant l'une des revendications 1 à 7, **caractérisés en ce que**
$R^4$ est choisi entre un reste cycloalkyle en $C_3$ à $C_8$, aryle ou hétéroaryle, chacun non substitué ou substitué une ou plusieurs fois ; ou un reste $-R^8-L-R^9$, avantageusement
$R^4$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle
ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinolyle, dioxolannyle, adamantyle, pyrimidinyle, quinolinyle, isoquinolinyle, phtalazinyle ou quinazolinyle, chacun non substitué ou substitué une ou plusieurs fois ; ou un reste $-R^8-L-R^9$ en particulier
$R^4$ est choisi entre un reste cyclopentyle, cyclohexyle, cycloheptyle, cyclooctyle, anthracényle, indolyle, naphtyle, benzofurannyle, benzothiazolyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois ; ou un reste $-R^8-L-R^9$

**18.** 4-aminocyclohexanols substitués suivant la revendication 17, **caractérisés en ce que**
$R^8$ est choisi entre un reste indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle
ou pyrimidyle, fluorényle, fluoranthényle, benzothiazolyle, benzotriazolyle ou benzo[1,2,5)thiazolyle
ou 1,2-dihydroacénaphtényle, pyridinyle, furannyle, benzofurannyle, pyrazolinonyle, oxopyrazolinonyle, pyrimidinyle, quinolinyle, isoquinolinyle, phtalazinyle ou quinazolinyle, chacun non substitué ou substitué une ou plusieurs fois,
L est choisi entre
-C(O)-NH-, -NH-C(O)-, -C(O)-O-, -O-C(O)-, -O-, -S- ou $-S(O)_2-$,
et/ou $R^9$ est choisi entre un reste indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5)thiazolyle ou 1,2-dihydroacénaphtényle, pyridinyle, furannyle, benzofurannyle, pyrazolinonyle, oxopyrazolinonyle, pyrimidinyle, quinolinyle, isoquinolinyle, phtalazinyle ou quinazolinyle, chacun non substitué ou substitué une ou plusieurs fois,
avantageusement
$R^8$ est choisi entre un reste indolyle, benzothiophényle, phényle, thiophényle, furyle, pyridyle, pyrrolyle, pyrazinyle ou pyrimidyle, chacun non substitué ou substitué une ou plusieurs fois,
L est choisi entre
-C(O)-NH-, -NH-C(O)-, -C(O)-O-, -O-C(O)- ou $-S(O)_2-$,
et/ou $R^9$ est choisi entre un reste indolyle, benzothiophényle, phényle, thiophényle, furyle, pyridyle, pyrrolyle, pyrazinyle ou pyrimidyle, chacun non substitué ou substitué une
ou plusieurs fois,
en particulier
$R^8$ représente un reste indolyle non substitué,
L représente

$-S(O)_2$,

et $R^9$ représente
un reste phényle non substitué.

**19.** 4-aminocyclohexanols substitués suivant l'une des revendications 1 à 7, **caractérisés en ce que**
$R^4$ est choisi entre un groupe $-CHR^6R^7$, $-CHR^6-CH_2R^7$, $-CHR^6-CH_2-CH_2R^7$,$-CHR^6-CH_2-CH_2-CH_2R^7$, $-C(Y)R^7$, $-C(Y)-CH_2R^7$, $-C(Y)-CH_2-CH_2R^7$ ou $C(Y)-CH_2-CH_2-CH_2R^7$
Y représentant O, S ou $H_2$,
avantageusement
$R^4$ est choisi entre $-CHR^6R^7$, $-CHR^6-CH_2R^7$, $-CHR^6-CH_2-CH_2R^7$, $-C(Y)R^7$, $-C(Y)-CH_2R^7$ ou $-C(Y)-CH_2-CH_2R^7$ Y représentant O ou S,
en particulier
$R^4$ est choisi entre $-CHR^6R^7$, $-CHR^6-CH_2R^7$, $-C(Y)R^7$, ou $-C(Y)-CH_2R^7$,

Y représentant O.

**20.** 4-aminocyclohexanols substitués suivant la revendication 19, **caractérisés en ce que**
$R^6$ est choisi entre H, un reste alkyle en $C_1$ à $C_4$, saturé ou non saturé, ramifié ou non ramifié, substitué une ou plusieurs fois ou non substitué ; ou un groupe C(O)O-(alkyle en $C_1$ à $C_4$), saturé ou non saturé, ramifié ou non ramifié, substitué une ou plusieurs fois ou non substitué ;
avantageusement
H, un reste alkyle en $C_1$ à $C_4$, saturé ou non saturé, ramifié ou non ramifié, substitué une ou plusieurs fois ou non substitué ; en particulier
H, $CH_3$ et $C_2H_5$.

**21.** 4-aminocyclohexanols substitués suivant la revendication 19, **caractérisés en ce que**
$R^7$ est choisi entre un reste cycloalkyle en $C_3$ à $C_8$, aryle ou hétéroaryle, chacun non substitué ou substitué une ou plusieurs fois ;
avantageusement
$R^7$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une
ou plusieurs fois ; en particulier
$R^7$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**22.** 4-aminocyclohexanols substitués suivant la revendication 8, **caractérisés en ce que**
$R^4$ est choisi entre des restes hétéroaryle, chacun non substitué ou substitué une ou plusieurs fois ; ou un groupe $-CHR^6R^7$, $-CHR^6-CH_2R^7$, $-CHR^6-CH_2-CH_2R^7$, $-CHR^5-CH_2-CH_2-CH_2R^7$, $-C(Y)R^7$, $-C(Y)-CH_2R^7$, $-C(Y)-CH_2-CH_2R^7$ ou $C(Y)-CH_2-CH_2-CH_2R^7$; ou $-R^8-L-R^9$ Y représentant $H_2$,
avantageusement
$R^4$ est choisi entre un reste indolyle, benzofurannyle, benzothiophényle, indanyle, benzodioxannyle, benzodioxolannyle, acénaphtyle, carbazolyle, thiophényle, furyle, pyridyle, pyrrolyle, pyrazinyle ou pyrimidyle, fluorényle, fluoranthényle, benzothiazolyle, benzotriazolyle ou benzo[1,2,5)thiazolyle ou 1,2-dihydroacénaphtényle, pyridinyle, furannyle, benzofurannyle, pyrazolinonyle, oxopyrazolinonyle, pyrimidinyle, quinolinyle, isoquinolinyle, phtalazinyle ou quinazolinyle, chacun non substitué ou substitué une ou plusieurs fois ; ou un groupe $-CHR^6R^7$, $-CHR^6-CH_2R^7$, $-CHR^6-CH_2-CH_2R^7$, $-C(Y)R^7$, $-C(Y)-CH_2R^7$ ou $-C(Y)-CH_2-CH_2R^7$, Y représentant $H_2$,
en particulier
$R^4$ est choisi entre un reste indolyle, benzofurannyle, benzothiophényle, benzothiazolyle, indanyle, benzodioxannyle, benzodioxolannyle, acénaphtyle, carbazolyle, thiophényle, furyle, pyridyle, pyrrolyle, pyrazinyle ou pyrimidyle, chacun non substitué ou substitué une ou plusieurs fois ; ou un groupe $-CHR^6R^7$, $-CHR^6-CH_2R^7$, $-C(Y)R^7$ ou $-C(Y)-CH_2R^7$, Y représentant $H_2$.

**23.** 4-aminocyclohexanols substitués suivant la revendication 22, **caractérisés en ce que**
$R^6$ est choisi entre H, un reste alkyle en $C_1$ à $C_4$, saturé ou non saturé, ramifié ou non ramifié, substitué une ou plusieurs fois ou non substitué,
avantageusement
H, un reste alkyle en $C_1$ à $C_4$, saturé ou non saturé, ramifié ou non ramifié, substitué une ou plusieurs fois ou non substitué ; en particulier
H, $CH_3$ et $C_2H_5$
et/ou
$R^7$ est choisi entre un reste indolyle, benzofurannyle, benzothiophényle, indanyle, benzodioxannyle, benzodioxolannyle, acénaphtyle, carbazolyle, thiophényle, furyle, pyridyle, pyrrolyle, pyrazinyle ou pyrimidyle, fluorényle, fluoranthényle, benzothiazolyle, benzotriazolyle ou benzo[1,2,5)thiazolyle
ou 1,2-dihydroacénaphtényle, pyridinyle, furannyle, benzofurannyle, pyrazolinonyle, oxopyrazolinonyle, pyrimidinyle, quinolinyle, isoquinolinyle, phtalazinyle ou quinazolinyle, chacun non substitué ou substitué une ou plusieurs fois ;
avantageusement

$R^7$ est choisi entre un reste indolyle, benzofurannyle, benzothiophényle, indanyle, benzodioxannyle, benzodioxolannyle, acénaphtyle, carbazolyle, thiophényle, furyle, pyridyle, pyrrolyle, pyrazinyle ou pyrimidyle, chacun non substitué ou substitué une ou plusieurs fois ; en particulier
$R^7$ est choisi entre un reste thiophényle, furyle, pyridyle, pyrrolyle, pyrazinyle ou pyrimidyle, chacun non substitué ou substitué une ou plusieurs fois.

**24.** 4-aminocyclohexanols substitués suivant la revendication 22, **caractérisés en ce que**
$R^8$ est choisi entre un reste indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinolyle, pyrimidinyle, quinolinyle, isoquinolinyle, phtalazinyle ou chinazolinyle, chacun non substitué ou substitué une ou plusieurs fois,
L est choisi entre -C(O)-NH-, -NH-C(O)-, -C(O)-O-, -O-C(O) ou $-S(O)_2-$,
et/ou $R^9$ est choisi entre un reste indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, pyrimidinyle, quinolinyle, isoquinolinyle, phtalozinyle ou quinazolinyle, chacun non substitué ou substitué une ou plusieurs fois, avantageusement,
$R^8$ est choisi entre un reste indolyle, benzothiophényle, phényle, thiophényle, furyle, pyridyle, pyrrolyle, pyrazinyle ou pyrimidyle, chacun non substitué ou substitué une
ou plusieurs fois,
L est choisi entre des groupes -C(O)-NH-, -NH-C(O)-, -C(O)-O-, -O-C(O)- ou $-S(O)_2-$
et/ou $R^9$ est choisi entre des restes indolyle, benzothiophényle, phényle, thiophényle, furyle, pyridyle, pyrrolyle, pyrazinyle ou pyrimidyle, chacun non substitué ou substitué une
ou plusieurs fois,
en particulier
$R^8$ est choisi entre
un reste indolyle non substitué,
L est un groupe

$$-S(O)_2-$$

et $R^9$ est un groupe phényle non substitué.

**25.** 4-aminocyclohexanols substitués suivant la revendication 9, **caractérisés en ce que**
$R^4$ est choisi entre $-CHR^6R^7$, $-CHR^6-CH_2R^7$ ou $-CHR^6-CH_2-CH_2R^7$ avantageusement
$R^4$ est choisi entre $-CHR^6R^7$ ou $-CHR^6-CH_2R^7$ en particulier
$R^4$ est choisi entre des groupes $-CHR^6R^7$.

**26.** 4-aminocyclohexanols substitués suivant la revendication 25, **caractérisés en ce que**
$R^6$ est choisi entre
un reste C(O)O-(alkyle en $C_1$ à $C_4$), saturé ou non saturé, ramifié ou non ramifié, substitué une ou plusieurs fois ou non substitué ;
avantageusement
un reste C(O)O-(alkyle en $C_1$ à $C_3$), saturé ou non saturé, ramifié ou non ramifié, substitué une ou plusieurs fois ou non substitué ; en particulier
$C(O)O-CH_3$ et $C(O)O-C_2H_5$
et/ou
$R^7$ est choisi entre un reste cycloalkyle en $C_3$ à $C_8$, aryle ou hétéroaryle, chacun non substitué ou substitué une ou plusieurs fois ;
avantageusement
$R^7$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une
ou plusieurs fois ; en particulier

$R^7$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**27.** 4-aminocyclohexanols substitués suivant la revendication 10, **caractérisés en ce que**
$R^4$ est choisi entre $-C(Y)R^7$, $-C(Y)-CH_2R^7$, $-C(Y)-CH_2-CH_2R^7$
ou $C(Y)-CH_2-CH_2-CH_2R^7$
Y représentant O
avantageusement
$R^4$ est choisi entre $-C(Y)R^7$, $-C(Y)-CH_2R^7$ ou $-C(Y)-CH_2-CH_2R^7$, Y représentant O
en particulier
$R^4$ est choisi entre $-C(Y)R^7$ ou $-C(Y)-CH_2R^7$,
Y représentant O.

**28.** 4-aminocyclohexanols substitués suivant la revendication 27, **caractérisés en ce que**
$R^7$ est choisi entre un reste cycloalkyle en $C_3$ à $C_8$, aryle ou hétéroaryle, chacun non substitué ou substitué une ou plusieurs fois ;
avantageusement
$R^7$ est choisi entre un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;
en particulier
$R^7$ est choisi entre un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**29.** 4-aminocyclohexanols substitués suivant l'une des revendications 1 à 28, **caractérisés en ce qu'**ils sont choisis dans le groupe suivant :

. 4-diméthylamino-1-(1-méthyl-1H-indole-2-yl)-4-phénylcyclohexanol
. 1-benzo[b]thiophène-2-yl-4-diméthylamino-4-phénylcyclohexanol
. 1-benzo[b]thiophène-3-yl-4-diméthylamino-4-phénylcyclohexanol
. 1-(1-benzènesulfonyl-1H-indole-2-yl)-4-diméthylamino-4-phénylcyclohexanol
. 1-benzofuranne-2-yl-4-diméthylamino-4-phénylcyclohexanol
. 1-benzothiazole-2-yl-4-diméthylamino-4-phénylcyclohexanol

le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates.

**30.** Médicament contenant au moins un 4-aminocyclohexanol substitué suivant l'une des revendications 1 à 29, éventuellement sous forme de son racémate, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme des acides ou des bases ou sous forme des sels, en particulier des sels physiologiquement acceptables, ou sous forme des produits de solvatation, en particulier des hydrates ; ainsi que le cas échéant des additifs et/ou des substances auxiliaires convenables et/ou le cas échéant d'autres substances actives.

**31.** Médicament suivant la revendication 30,
**caractérisé en ce qu'**il contient encore, à côté d'au moins un 4-aminocyclohexanol substitué, un opiacé, avantageusement un opiacé très actif, en particulier la morphine, ou un anesthésique, avantageusement l'hexobarbital ou l'halothane.

**32.** Utilisation d'un 4-aminocyclohexanol substitué suivant l'une des revendications 1 à 29, éventuellement sous forme de son racémate, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme des acides ou des bases ou sous forme des sels, en particulier des sels physiologiquement acceptables, ou sous forme des produits de solvatation, en particulier des hydrates ; pour la préparation d'un médicament destiné au traitement de la douleur, en particulier d'une douleur aiguë, viscérale, neuropathique ou chronique, de la migraine ou de troubles locomoteurs ; comme anti-convulsif ou comme myorelaxant.

**33.** Utilisation d'un 4-aminocyclohexanol substitué de formule générale I,

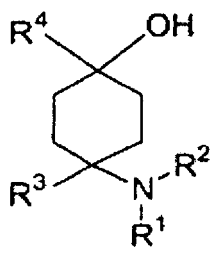

I

dans laquelle
$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ; $R^1$ et $R^2$ ne pouvant pas représenter tous deux H,
ou bien les restes $R^1$ et $R^2$ forment ensemble un noyau et représentent $CH_2CH_2OCH_2CH_2$, $CH_2CH_2NR^5CH_2CH_2$ ou $(CH_2)_{3-6}$,
$R^5$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ;
$R^3$ est choisi entre un reste aryle ou un reste hétéroaryle, chacun non substitué ou substitué une ou plusieurs fois ;
$R^4$ est choisi entre un reste cycloalkyle en $C_3$ à $C_8$, aryle ou hétéroaryle, chacun non substitué ou substitué une ou plusieurs fois ; un groupe $-CHR^6R^7$, $-CHR^6-CH_2R^7$,- $CHR^6-CH_2-CH_2R^7$, $-CHR^6-CH_2-CH_2-CH_2R^7$, $-C(Y)R^7$, -C (Y)-$CH_2R^7$, -C (Y) -$CH_2-CH_2R^7$ ou -C(Y)-$CH_2-CH_2-CH_2R^7$; ou $-R^8-L-R^9$ ,
Y représentant O, S ou $H_2$,
$R^6$ étant choisi entre H, un reste alkyle en $C_1$ à $C_7$, saturé ou non saturé, ramifié ou non ramifié, substitué une ou plusieurs fois ou non substitué ; ou un groupe C(O)O-(alkyle en $C_1$ à $C_6$), saturé ou non saturé, ramifié ou non ramifié, substitué une ou plusieurs fois ou non substitué ;
et $R^7$ étant choisi entre H ; un reste cycloalkyle en $C_3$ à $C_8$, aryle ou hétéroaryle, chacun non substitué ou substitué une ou plusieurs fois,
$R^8$ étant choisi entre un reste aryle ou hétéroaryle, chacun non substitué ou substitué une ou plusieurs fois,
L étant choisi entre
-C(O)-NH-, -NH-C(O)-, -C(O)-O-, -O-C(O)-, -O-, -S- ou $-S(O)_2-$
$R^9$ étant choisi entre
un reste aryle ou hétéroaryle, chacun non substitué ou substitué une ou plusieurs fois,
"aryle ou hétéroaryle substitué une ou plusieurs fois" désignant un reste "aryle ou hétéroaryle substitué une ou plusieurs fois avec $R^{82}$, $OR^{82}$, un halogène, un groupe $CF_3$, CN, $NO_2$, $NR^{83}R^{84}$, alkyle en $C_1$ à $C_6$, alkoxy en $C_1$ à $C_6$, cycloalkoxy en $C_3$ à $C_8$, cycloalkyle en $C_3$ à $C_8$ ou alkylène en $C_2$ à $C_6$",
$R^{82}$ représentant H, un reste alkyle en $C_1$ à $C_{10}$, aryle, hétéroaryle ou un reste aryle ou hétéroaryle lié par l'intermédiaire d'un groupe alkyle en $C_1$ à $C_3$ saturé ou non saturé ou d'un groupe alkylène en $C_1$ à $C_3$, ces restes aryle

et hétéroaryle ne pouvant pas être substitués eux-mêmes avec des restes aryle ou hétéroaryle ; $R^{83}$ et $R^{84}$, identiques ou différents, représentent H, un reste alkyle en $C_1$ à $C_{10}$, aryle, hétéroaryle ou un reste aryle ou hétéroaryle lié par l'intermédiaire d'un groupe alkyle en $C_1$ à $C_3$, saturé ou non saturé, ou d'un groupe alkylène en $C_1$ à $C_3$, ces restes aryle et hétéroaryle ne pouvant pas eux-mêmes être substitués avec des restes aryle ou hétéroaryle, ou bien les restes $R^{83}$ et $R^{84}$ représentent ensemble un groupe $CH_2CH_2OCH_2CH_2$, $CH_2CH_2NR^{85}CH_2CH_2$ ou $(-CH_2)_{3-6}$, et le reste $R^{85}$ représente H, un reste alkyle en $C_1$ à $C_{10}$, aryle ou hétéroaryle ou un reste aryle ou hétéroaryle lié par l'intermédiaire d'un groupe alkyle en $C_1$ à $C_3$, saturé ou non saturé, ou d'un groupe alkylène en $C_1$ à $C_3$, ces restes aryle et hétéroaryle ne pouvant pas être substitués eux-mêmes avec des restes aryle ou hétéroaryle ; "cycloalkyle substitué une ou plusieurs fois" désigne "un reste cycloalkyle substitué une ou plusieurs fois avec F, Cl, Br, I, $NH_2$, SH, OH, O(alkyle en $C_1$ à $C_3$) ou alkyle en $C_1$ à $C_3$" ; et

"alkyle substitué une ou plusieurs fois" désigne "un groupe alkyle substitué une ou plusieurs fois avec F, Cl, Br, I, $NH_2$, SH ou OH",

éventuellement sous forme de son racémate, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme des acides ou des bases ou sous forme des sels, en particulier des sels physiologiquement acceptables, ou sous forme des produits de solvatation, en particulier des hydrates ; pour la préparation d'un médicament destiné au traitement d'états d'angoisse, du stress et de syndromes liés à un stress, de dépressions, de l'épilepsie, de la maladie d'Alzheimer, de la démence sénile, de dysfonctionnements cognitifs généraux, de difficultés d'apprentissage et de mémoire (agent nootrope), de phénomènes de privation, d'abus et/ou de dépendance à l'égard de l'alcool et/ou des drogues et/ou des médicaments, de dysfonctionnements sexuels, de maladies cardiovasculaires, de l'hypotension, de l'hypertension, des acouphènes, du prurit, de la surdité, de l'insuffisance de motilité intestinale, de troubles de l'absorption de nourriture, de l'anorexie, de l'obésité, de la diarrhée, de la cachexie, de l'incontinence d'urine et comme anti-tussif ou anesthésique ou de la co-administration lors d'un traitement avec un analgésique opiacé ou avec un anesthésique, en vue de la diurèse ou de l'anti-natriurèse et/ou en vue de l'anxiolyse.

**34.** Procédé de production d'un 4-aminocyclohexanol substitué suivant l'une des revendications 1 à 29, comprenant les étapes suivantes :

a. une cyclohexane-1,4-dione protégée avec les groupes $S^1$ et $S^2$, répondant à la formule II, est amenée à réagir en présence d'un composé de formule $HNR^{01}R^{02}$ avec un cyanure, avantageusement le cyanure de potassium, pour former un dérivé de 1-amino-4-oxo-cyclohexanecarbonitrile N-substitué protégé répondant à la formule III :

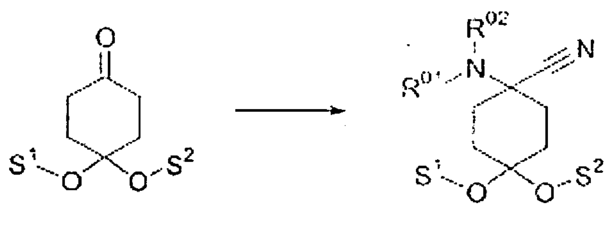

II III

on conduit ensuite éventuellement dans un ordre quelconque et le cas échéant de façon répétée une acylation, une alkylation ou une sulfonation et/ou dans le cas de composés pour lesquels $R^{01}$ et/ou $R^{02}$ et/ou $R^{06}$ représentent un atome d'hydrogène protégé avec un groupe protecteur, on élimine au moins une fois un groupe protecteur et on conduit éventuellement une acylation, une alkylation ou une sulfonation et/ou dans le cas de composés pour lesquels $R^{01}$ et/ou $R^{02}$ et/ou $R^{06}$ représentent l'hydrogène, on introduit au moins une fois un groupe protecteur et on conduit éventuellement une acylation, une alkylation ou une sulfonation,

b. l'aminonitrile répondant à la formule III est amenée à réagir avec des réactifs organométalliques, avantageusement des réactifs de Grignard ou des réactifs organiques de lithium, de formule métal-$R^3$, de manière à produire un composé répondant à la formule IVa :

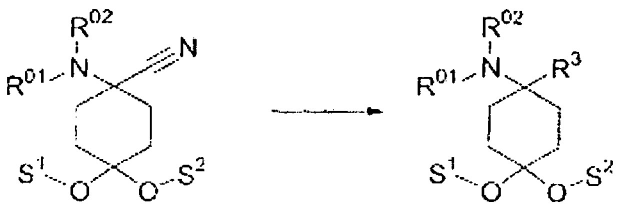

III IVa

on conduit ensuite éventuellement dans un ordre quelconque et le cas échéant de façon répétée une acylation, une alkylation ou une sulfonation et/ou dans le cas de composés pour lesquels $R^{01}$ et/ou $R^{02}$ et/ou $R^{06}$ représentent un atome d'hydrogène protégé avec un groupe protecteur, on élimine au moins une fois un groupe protecteur et on conduit éventuellement une acylation, une alkylation ou une sulfonation et/ou dans le cas de composés pour lesquels $R^{01}$ et/ou $R^{02}$ et/ou $R^{06}$ représentent l'hydrogène, on introduit au moins une fois un groupe protecteur et on conduit éventuellement une acylation, une alkylation ou une sulfonation,

c. les groupes protecteurs $S^1$ et $S^2$ portés par le composé de formule IVa selon la formule III sont éliminés, de manière à produire un dérivé de 4-aminocyclohexanone 4-substitué répondant à la formule IV :

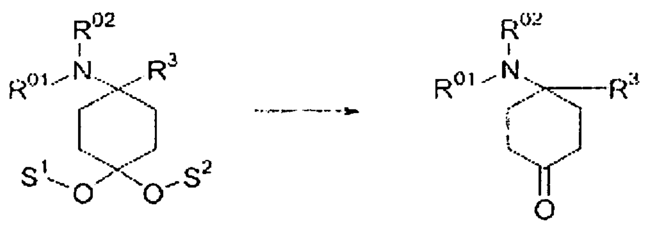

IVa IV

on conduit ensuite éventuellement dans un ordre quelconque et le cas échéant de façon répétée une acylation, une alkylation ou une sulfonation et/ou dans le cas de composés pour lesquels $R^{01}$ et/ou $R^{02}$ et/ou $R^{06}$ représentent un atome d'hydrogène protégé avec un groupe protecteur, on élimine au moins une fois un groupe protecteur et on conduit éventuellement une acylation, une alkylation ou une sulfonation et/ou dans le cas de composés pour lesquels $R^{01}$ et/ou $R^{02}$ et/ou $R^{06}$ représentent l'hydrogène, on introduit au moins une fois un groupe protecteur et on conduit éventuellement une acylation, une alkylation ou une sulfonation,

d. le dérivé de 4-aminocyclohexanone 4-substitué répondant à la formule IV est amené à réagir avec des réactifs organométalliques, avantageusement des réactifs de Grignard ou des réactifs organiques de lithium, de formule métal-$R^{04}$, afin de produire un composé répondant à la formule V :

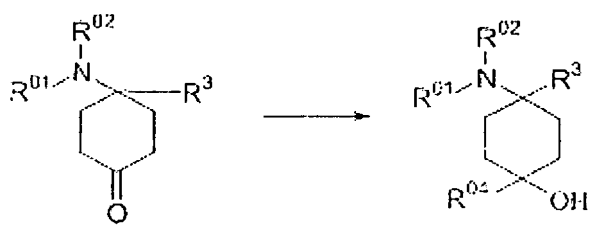

IV V

on conduit ensuite éventuellement dans un ordre quelconque et le cas échéant de façon répétée une acylation, une alkylation ou une sulfonation et/ou dans le cas de composés pour lesquels $R^{01}$ et/ou $R^{02}$ et/ou $R^{04}$ et/ou $R^{05}$ et/ou $R^{06}$ représentent un atome d'hydrogène protégé avec un groupe protecteur, on élimine au moins une fois un groupe protecteur et on conduit éventuellement une acylation, une alkylation

ou une sulfonation et/ou dans le cas de composés pour lesquels $R^{01}$ et/ou $R^{02}$ et/ou $R^{04}$ et/ou $R^{05}$ et/ou $R^{06}$ représentent l'hydrogène, on introduit au moins une fois un groupe protecteur et on conduit éventuellement une acylation, une alkylation ou une sulfonation, jusqu'à ce qu'un composé de formule I ait été produit, $R^1$, $R^2$, $R^3$ et $R^4$ ayant la définition indiquée dans la revendication 1, et

$R^{01}$ et $R^{02}$ sont choisis indépendamment l'un de l'autre entre H ; H pourvu d'un groupe protecteur ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ;

ou bien les restes $R^{01}$ et $R^{02}$ forment ensemble un noyau et représentent $CH_2CH_2OCH_2CH_2$, $CH_2CH_2NR^{05}CH_2CH_2$ ou $(CH_2)_{3-6}$,

$R^{05}$ étant choisi entre H ; H pourvu d'un groupe protecteur, un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou bien un reste aryle, cycloalkyle en $C_3$ à $C_8$ ou hétéroaryle lié par l'intermédiaire d'un groupe alkylène en $C_1$ à $C_3$, chacun substitué une ou plusieurs fois ou non substitué ;

$R^{04}$ est choisi entre H, H pourvu d'un groupe protecteur ; un reste cycloalkyle en $C_3$ à $C_8$, aryle ou hétéroaryle, chacun non substitué ou substitué une ou plusieurs fois ; un groupe $-CHR^6R^7$, $-CHR^6-CH_2R^7$, $-CHR^6-CH_2-CH_2R^7$, $-CHR^6CH_2-CH_2-CH_2R^7$, $-C(Y)R^7$, $-C(Y)-$, $CH_2R^7$, $-C (Y) -CH_2-CH_2R^7$ ou $-C(Y)-CH_2-CH_2-CH_2R^7$; ou $-R^8-L-R^9$ ,

Y représentant O, S ou $H_2$,

$R^6$ étant choisi entre H, un reste alkyle en $C_1$ à $C_7$, saturé ou non saturé, ramifié ou non ramifié, substitué une ou plusieurs fois ou non substitué ; et

$R^7$ étant choisi entre H ; un reste cycloalkyle en $C_3$ à $C_8$, aryle ou hétéroaryle, chacun non substitué ou substitué une ou plusieurs fois,

$R^8$ étant choisi entre un reste aryle ou hétéroaryle, chacun non substitué ou substitué une ou plusieurs fois,

L étant choisi entre

-C(O)-NH-, -NH-C(O)-, -C(O)-O-, -O-C(O)-, -O-, -S- ou $-S(O)_2-$

$R^9$ étant choisi entre

un reste aryle ou hétéroaryle, chacun non substitué ou substitué une ou plusieurs fois, et $S^1$ et $S^2$ sont choisis indépendamment l'un de l'autre parmi les groupes protecteurs ou représentent ensemble un groupe protecteur, avantageusement un groupe monoacétal.

**35.** Procédé de production d'un 4-aminocyclohexanol substitué suivant la revendication 34, **caractérisé en ce que** les groupes protecteurs au niveau de H pour $R^{01}$, $R^{02}$, $R^{04}$ et/ou $R^{05}$ sont choisis entre des groupes alkyle, le groupe benzyle ou des groupes carbamate, par exemple FMOC, Z ou Boc.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS2839891 A
- US 4366172 A


### In der Beschreibung aufgeführte Nicht-Patentliteratur


- **Meunier et al.** *Nature,* 1995, vol. 377, 532-535
- **Mollereau et al.** *FEBS Letters,* 1994, vol. 341, 33-38
- **Darland et al.** *Trends in Neurosciences,* 1998, vol. 21, 215-221
- **Ardati et al.** *Mol. Pharmacol.,* vol. 51, 816-824
- **Matthes et al.** *Mol. Pharmacol.,* 1996, vol. 50, 447-450
- **Vaughan et al.** *Br. J. Pharmacol.,* 1996, vol. 117, 1609-1611
- **Conner et al.** *Br. J. Pharmacol.,* 1996, vol. 118, 205-207
- **Knoflach et al.** *J. Neuroscience,* 1996, vol. 16, 6657-6664
- **scheid et al.** *Science,* 1995, vol. 270, 792-794
- **Hara et al.** *Br. J. Pharmacol.,* 1997, vol. 121, 401-408
- **Mogil et al.** *Neurosci. Letters,* 1996, vol. 214, 131-134
- *Neuroscience,* 1996, vol. 75, 333-337
- **Jenck et al.** *Proc. Natl. Acad. Sci. USA,* 1997, vol. 94, 14854-14858
- **Shu et al.** *Neuropeptides,* 1998, vol. 32, 567-571
- **Faber et al.** *Br. J. Pharmacol.,* 1996, vol. 119, 189-190
- **King et al.** *Neurosci. Lett.,* 1997, vol. 223, 113-116
- **Xu et al.** *NeuroReport,* 1996, vol. 7, 2092-2094
- **Yamamoto et al.** *Neuroscience,* 1997, vol. 81, 249-254
- **Yamamoto ; Nozaki-Taguchi.** *Anesthesiology,* 1997, 87
- **Abdulla ; Smith.** *J. Neurosci.,* 1998, vol. 18, 9685-9694
- **Sandin et al.** *Eur. J. Neurosci.,* 1997, vol. 9, 194-197
- **Manabe et al.** *Nature,* 1997, vol. 394, 577-581
- **Nishi et al.** *EMBO J.,* 1997, vol. 16, 1858-1864
- **Pomonis et al.** *NeuroReport,* 1996, vol. 8, 369-371
- **Gumusel et al.** *Life Sci.,* 1997, vol. 60, 141-145
- **Campion ; Kadowitz.** *Biochem. Biophys. Res. Comm.,* 1997, vol. 234, 309-312
- **Gutiérrez et al.** *Abstract 536.18, Society for Neuroscience,* 07. November 1998, vol. 24
- **Kapista et al.** *Life Sciences,* 1997, vol. 60
- **Calo et al.** *Br.J. Pharmacol.,* 2000, vol. 129, 1261-1283
- **D. Lednicer ; P.F. von Voightlander.** *J. Med. Chem.,* 1979, vol. 22, 1157
- **D. Lednicer ; P.F. von Voightlander ; D.E. Emmert.** *J. Med. Chem.,* 1980, vol. 23, 424
- **D. Lednicer ; P.F. von Voightlander ; D.E. Emmert.** *J. Med. Chem.,* 1981, vol. 24, 404
- **D. Lednicer ; P.F. von Voightlander ; D.E. Emmert.** *J. Med. Chem.,* 1981, vol. 24, 340
- Endogenous and Exogenous Opiate Agonists and Antagonists. **P.F. VonVoightlander ; D. Lednicer ; R.A. Lewis ; D.D. Gay.** Proc. Int. Narc. Res. Club Conf. Pergamon, 1979, 17-21
- **Kamenka et al.** *EurJ.Med.Chem.Chim.Ther.,* 1984, vol. 19 (3), 255-260
- **Rao M.N.A. ; Rao S.C.** *Indian Drugs,* 1985, vol. 22 (5), 252-257
- **Ardati et al.** *Mol. Pharmacol.,* 1997, vol. 51, 816-824
- **D'Amour ; Smith.** *J. Pharm. Exp. Ther.,* 1941, vol. 72, 74 79